(19) Europäisches Patentamt
European Patent Office
Office européen des brevets

(11) **EP 1 509 543 B1**

(12) **FASCICULE DE BREVET EUROPEEN**

(45) Date de publication et mention de la délivrance du brevet:
**17.09.2008 Bulletin 2008/38**

(21) Numéro de dépôt: **03747137.2**

(22) Date de dépôt: **04.04.2003**

(51) Int Cl.:
***C07K 14/025*** *(2006.01)*

(86) Numéro de dépôt international:
**PCT/FR2003/001082**

(87) Numéro de publication internationale:
**WO 2003/090667 (06.11.2003 Gazette 2003/45)**

(54) **COMPOSITION IMMUNOGENE OU VACCINALE NON IMMUNOSUPPRESSIVE, COMPRENANT UNE PROTEINE E7 MUTEE DU VIRUS HPV-16**

NICHTIMMUNSUPPRESSIVE IMMUNOGENE ODER VAKZINZUSAMMENSETZUNG ENTHALTEND EIN MUTIERTES E7-PROTEIN VOM HUMANEN PAPILLOMA-VIRUS HPV-16

NON-IMMUNOSUPPRESSIVE IMUNOGENIC OR VACCINE COMPOSITION COMPRISING A MUTATED E7 PROTEIN OF THE HPV-16 VIRUS

(84) Etats contractants désignés:
**AT BE BG CH CY CZ DE DK EE ES FI FR GB GR HU IE IT LI LU MC NL PT RO SE SI SK TR**

(30) Priorité: **24.04.2002 FR 0205173**

(43) Date de publication de la demande:
**02.03.2005 Bulletin 2005/09**

(73) Titulaire: **NEOVACS**
**75016 Paris (FR)**

(72) Inventeurs:
- **ZAGURY, Daniel**
  **F-75007 Paris (FR)**
- **LE BUANEC, Hélène**
  **F-75005 Paris (FR)**
- **BIZZINI, Bernard**
  **F-81000 Albi (FR)**

(74) Mandataire: **Catherine, Alain et al**
**Cabinet Harlé & Phélip**
**7, rue de Madrid**
**75008 Paris (FR)**

(56) Documents cités:

| | |
|---|---|
| **EP-A- 0 451 550** | **WO-A-01/14416** |
| **WO-A-96/19496** | **WO-A-99/10375** |
| **FR-A- 2 794 371** | **US-B1- 6 235 523** |

EP 1 509 543 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

## DOMAINE DE L'INVENTION

La présente invention se rapporte au domaine de la prévention ou du traitement de certains cancers associés à une infection par le Papillomavirus de type HPV-16.

## ART ANTERIEUR

Plusieurs espèces de papillomavirus humain, ou HPV, sont considérées aujourd'hui comme les agents causals de cancers. En particulier, les HPV de types 16, 18, 31, 33 et 51 sont fréquemment associés aux cancers ano-génitaux, y compris le cancer du col utérin de la femme.

Plus de 50% des carcinomes squameux du col utérin sont associés au papillomavirus HPV de type 16.

Les oncoprotéines virales E6 et E7 de HPV sont fortement impliquées dans les mécanismes moléculaires par lesquels ces virus contribuent au développement de ces cancers. Ces deux oncoprotéines agissent en inactivant les régulateurs du cycle cellulaire que constituent la protéine p53 et la protéine du rétinoblastome, provoquant ainsi l'événement initiateur de la progression à étapes multiples vers le cancer.

En général, les tumeurs malignes sont constituées de cellules cancéreuses porteuses d'antigènes associés (TAA) ou d'antigènes spécifiques (TSA) et d'un micro-environnement stromal particulier, caractérisées par une hypervascularisation, ou néoangiogénèse, qui peut être accompagnée d'une paralysie des cellules immunitaires, c'est-à-dire à un état d'immunosuppression. Initialement, l'immunosuppression reste localisée au niveau de la tumeur, puisque l'individu est encore capable de se défendre contre les autres agressions telles que des infections.

Cependant, avec la dissémination métastatique, l'immunosuppression peut s'étendre et se généraliser, comme l'atteste la fragilité aux infections du cancéreux au stade terminal . Cette immunosuppression met en jeu des facteurs paralysants qui sont produits par les cellules cancéreuses ou par les cellules de leur environnement. La paralysie locale des cellules du système immunitaire, ou immunosuppression, représente donc une arme majeure des cellules cancéreuses qui leur permet d'échapper au système immunitaire de l'hôte.

Cet état d'immunosuppression a aussi été observé dans les cancers provoqués par une infection par HPV-16. Cette immunosuppression constitue aujourd'hui un obstacle technique majeur dans la mise au point de compositions vaccinales préventives ou curatives à l'encontre des cancers provoqués par une infection par HPV-16. En effet, les stratégies vaccinales anti-HPV-16 actuelles privilégient l'induction d'une prolifération de cellules T-cytotoxiques reconnaissant spécifiquement certains antigènes produits par HPV-16, en particulier les protéines E6 et E7, lorsqu'ils sont associés aux antigènes de classe I du complexe majeur d'histocompatibilité (MHC) à la surface membranaire des cellules cancéreuses infectées.

Certains auteurs ont associé l'immunosuppression observée dans des cancers provoqués par HPV-16 à l'observation de niveaux d'expression réduits des molécules de classe I du MHC à la surface des cellules cancéreuses du col utérin (Cruz et al., 1999), ou encore à une réduction de l'expression de la chaîne delta du récepteur des cellules T (TCR) chez des patients exprimant une dysplasie pré-néoplastique (Muderspach et al., 2000). D'autres auteurs ont associé l'état d'immunosuppression à un défaut de réponse des cellules T à d'éventuels signaux produits par les cellules tumorales et transmis aux cellules immunitaires en provoquant leur apoptose, par exemple du fait d'une interaction Fas/Fas-ligand (Schoell et al., 1999).

D'autres auteurs ont observé que l'immunosuppression associée à un cancer provoqué par une infection par HPV était concomitante à une augmentation de IL-10 (El Sherif et al. 2001 ; Giannini et al. 1998 ; Giannini et al. 2002) et de TGF-bêta-1 (Giannini et al. 1998) ainsi qu'à une réduction de l'IFN-gamma sous-épithélial (El Sherif et al. , 2001).

Des travaux antérieurs des inventeurs relatés dans la demande internationale publiée sous le n˚WO 00/03732 , ont montré que, dans le cas du cancer du col utérin provoqué par HPV, la protéine E7 du papillomavirus était impliquée dans une immunosuppression locale au niveau des tumeurs ou de cellules infectées.

L'immunosuppression induite par la protéine E7 a été caractérisée par l'inhibition de la prolifération des cellules T stimulées par le PPD ou le toxoïde tétanique, l'inhibition de la prolifération des cellules T stimulées par des cellules allogéniques, la surproduction d'IFN-$\alpha$ (cytokine immunosuppresive ) par les cellules présentant l'antigène (APC). Afin de réduire ou de bloquer l'immunosuppression induite par la protéine E7 soluble de HPV, il a été proposé l'utilisation de dérivés non toxiques de la protéine E7 comme antigène en vue de l'induction d'anticorps dirigés spécifiquement contre la protéine E7 extracellulaire. En vue de fabriquer des composés immunogènes dérivés de E7 dépourvus des effets délétères de la protéine native, il a été proposé de modifier la protéine E7 native, soit par modification chimique, soit par modification génétique, notamment en opérant des insertions, des délétions ou des substitutions de résidus d'acides aminés destinées à diminuer ou supprimer les sites fonctionnels délétères de la protéine native. Dans ce contexte, il a été décrit une protéine E7 modifiée chimiquement, dénuée d'activité immunosuppressive, après traitement de la protéine E7 native par le glutaraldéhyde.

Les modifications chimiques proposées pour réduire ou bloquer les propriétés d'immunosuppression de la protéine E7 native, telles que décrites dans l'état de la technique, visent à ajouter un groupe chimique au squelette carboné de la protéine E7 native, par couplage d'une fonction réactive de l'un des acides aminés de cette protéine avec une fonction aldéhyde, carboxamide ou maléimide, sans altération de la séquence en acides aminés de la protéine native, c'est-à-dire sans apporter de modifications significatives à la structure primaire de la protéine E7.

De fait, les diverses compositions immunogènes anti-E7 décrites dans l'état de la technique, en vue de la réalisation de préparations vaccinales préventives ou curatives à l'encontre de cancers provoqués par une infection par HPV-16, qui visent prioritairement l'induction d'une réponse immunitaire par la production de lymphocytes cytotoxiques (CTL) spécifiques de la protéine E7 exprimée à la surface des cellules infectées, contiennent, en tant qu'antigène, soit la protéine E7 complète, éventuellement modifiée chimiquement (Gérard et al., 2001) soit des peptides dérivés de la protéine E7 (Muderspach et al., 2000 ; Schoell, 1999 - précités). Dans tous les cas, y compris lorsque des peptides dérivés de la protéine E7 native sont utilisés, il n'a pas été introduit de modification dans la séquence d'acides aminés de la protéine native, et par conséquent dans la structure primaire d'acides aminés initiale. Cela s'explique simplement par la volonté de conserver intacts les épitopes d'intérêt de la protéine E7 native afin de favoriser l'induction d'une réponse immunitaire, en particulier la production de cellules CTL, capables de reconnaître spécifiquement et efficacement la protéine E7 native produite par les cellules infectées.

Et même lorsqu'une réorganisation de la structure primaire en acides aminés de la protéine E7 est envisagée, en vue de la production d'une protéine immunogène ayant des propriétés carcinogènes, ou transformantes réduites, il est porté une grande attention à la conservation d'au moins une copie complète de la séquence d'acides aminés de la protéine E7 native au sein du composé immunogène réarrangé, afin de maintenir présent, au sein dudit composé immunogène, l'ensemble des épitopes potentiels de la protéine E7 native.

Ainsi, Osen et al. (2001) ont décrit la construction d'un ADN codant un composé immunogène susceptible d'induire la production de lymphocytes cytotoxiques (CTL) reconnaissant spécifiquement la protéine E7 native exprimée à la surface des cellules cancéreuses infectées par HPV-16, dans une stratégie vaccinale anti-HPV avec de l'ADN, et non directement avec un composé immunogène peptidique. Afin d'éviter tout événement de recombinaison qui pourrait aboutir à une surproduction de protéine E7 native, ou tout au moins d'une protéine recombinée possédant les propriétés carcinogènes de la protéine E7 native, ces auteurs ont construit un ADN codant pour une protéine immunogène dans laquelle l'ensemble des épitopes de la protéine E7 native est représenté, bien que chacun des domaines peptidiques, respectivement (i) domaine 1-10, (ii) domaine 11-40, (iii) domaine 41-70 et (iv) domaine 71-98, aient été intervertis dans le composé peptidique immunogène final. Ces auteurs insistent sur la nécessité de produire un composé peptidique immunogène ayant conservé tous les épitopes CTL potentiels de la protéine E7 native, tout en ayant simultanément perdu ses propriétés de transformation cancéreuses des cellules. En dernier lieu, ces auteurs suggèrent d'accroître encore l'innocuité du composé peptidique immunogène réarrangé, en ce qui concerne son pouvoir cancérigène, en supprimant le domaine de liaison de la protéine E7 native à la protéine pRB du rétinoblastome, allant de l'acide aminé 21 à l'acide aminé 26 de E7, responsable de l'activité carcinogène de la protéine E7 native. Toutefois, ce mode de réalisation d'un composé peptidique immunogène non carcinogène n'a pas été concrètement réalisé.

## DESCRIPTION DE L'INVENTION

De manière surprenante, il a désormais été montré selon l'invention qu'il pouvait être obtenu, par mutagenèse dirigée de l'ADN codant la protéine E7 native, une protéine mutée possédant une capacité identique, voire une capacité améliorée, à induire une réponse immunitaire spécifique à l'encontre de la protéine E7 native, et dans laquelle ont été bloquées les propriétés d'immunosuppression de la protéine E7 native.

Il a ainsi été montré pour la première fois selon l'invention, de manière surprenante, qu'un fragment peptidique localisé dans la région allant de l'acide aminé en position 20 (Thr ou T) jusqu'à l'acide aminé en position 29 (Asn ou N) de la protéine E7 native du Papillomavirus HPV-16 était responsable de l'activité immunosuppressive de cette protéine.

Aux fins de la présente description, une protéine qui diffère d'au moins un acide aminé, par rapport à la protéine E7 native de HPV-16 de séquence SEQ ID N°3, est désignée comme une protéine E7 « mutée » selon l'invention, lorsque la ou les différences en acides aminés sont localisées dans la région peptidique correspondante 20-29 de la protéine E7 native de séquence SEQ ID N°3.

En particulier, il a été montré selon l'invention qu'une protéine E7 mutée qui comprend la substitution d'au moins un acide aminé, de préférence d'au moins deux acides aminés, par rapport à la séquence SEQ ID N°3 de la protéine E7 native, a perdu la propriété d'induire l'immunosuppression qui avait été rapportée antérieurement pour la protéine E7 native. On a ainsi montré qu'une protéine E7 mutée comprenant la substitution du résidu acide cystéine en position 24 par un résidu de glycine (CYS24GLY) et la substitution du résidu d'acide glutamique en position 26 par un résidu glutamine (GLU26GLN), par rapport à la séquence SEQ ID N°3 de la protéine E7 native, a perdu la propriété immunosuppressive de la protéine E7 native. De même, on a montré qu'une protéine E7 mutée comprenant la substitution du résidu cystéine en position 24 par un résidu de sérine (CYS24SER) et la substitution du résidu d'acide glutamique

en position 26 par un résidu de glutamine (GLU26GLN), par rapport à la séquence SEQ ID N°3 de la protéine E7 native, a perdu la propriété immunosuppressive de la protéine E7 native.

On a aussi montré selon l'invention qu'une protéine E7 mutée qui comprend, par rapport à la séquence d'acides aminés SEQ ID N°3 de la protéine E7 native, une délétion d'au moins quatre, et de préférence une délétion de quatre, cinq, six, sept, huit, neuf ou dix acides aminés consécutifs dans la région polypeptidique correspondante de la séquence SEQ ID N°3 allant de l'acide aminé en position 20 jusqu'à l'acide aminé en position 29, selon la numérotation de la séquence SEQ ID N°3, a perdu la propriété immunosuppressive observée pour la protéine E7 native.

En particulier, on a montré que la protéine E7 mutée comprenant la délétion du fragment peptidique 21(Asp ou D) à 26( Glu ou E), selon la numérotation de la séquence SEQ ID N°3, a perdu la propriété immunosuppressive observée pour la protéine E7 native. La protéine E7 mutée dont la région correspondant aux acides aminés 21 à 26 de la protéine E7 native est délétée est aussi désignée comme la protéine E7Δ21-26 aux fins de la présente description.

En tant que telle, la protéine E7Δ21-26, produite par recombinaison génétique, a été décrite par D'Anna et al. (2001, J. Natl. Cancer Inst. Vol. 93 (24): 1843-1851).

De même, on a montré que la protéine E7 mutée comprenant la délétion du fragment peptidique 21(Asp ou D) à 25( Tyr ou Y), selon la numérotation de la séquence SEQ ID N°3, a perdu la propriété immunosuppressive observée pour la protéine E7 native. La protéine E7 mutée dont la région correspondant aux acides aminés 21 à 25 de la protéine E7 native est délétée est aussi désignée comme la protéine E7Δ21-25 aux fins de la présente description.

Il est donc montré selon l'invention que de simples substitutions d'acides aminés dans la région peptidique 20-29 de la protéine E7 native permettent de bloquer l'activité immunosuppressive observée pour cette protéine. Ainsi, des altérations minimes de la structure primaire de la protéine E7 native, dans la région peptidique 20-29, détruisent les propriétés immunosuppressives caractéristiques de la protéine E7 native.

Selon l'invention, une protéine est « immunosuppressive » lorsque cette protéine inhibe d'au minimum 60%, et de préférence d'au moins 70%, la prolifération *in vitro* de cellules mononucléées provenant du sang périphérique humain d'un individu vacciné contre le tétanos (Antigène tétanos toxoïde) et/ou la diphtérie ( antigène du type « tuberculin-Purified Protein Derivative » ou « PPD »), ladite prolifération des cellules mononucléées étant induite par pré-incubation de ces cellules avec (i) l'antigène PPD, (ii) avec l'antigène tetanus toxoïde, (iii) avec une association de l'antigène PPD et de l'antigène tetanus toxoïde, ou encore (iv) avec des cellules allogéniques vis-à-vis desdites cellules mononucléées. Dans ce test, la protéine potentiellement immunosuppressive est utilisée à la concentration induisant la valeur maximale d'inhibition de prolifération, selon une courbe d'effet-dose. Une illustration d'un tel test donnée à l'exemple 6.

Egalement, une protéine est « immunosuppressive » lorsque cette protéine induit un niveau de production *in vitro* anormal de cytokines immunosuppressives, respectivement l'IL-10 par les cellules mononucléées du sang périphérique, et l'IFN-$\alpha$ ou l'IL-10 par les cellules présentant l'antigène (APC).

De plus, comme cela sera précisé plus loin dans la description, et comme cela est illustré dans les exemples, les protéines E7 mutées ci-dessus possèdent toutes en commun, en plus de leur caractère non immunosuppressif, également la capacité de stimuler la production d'anticorps « neutralisants » vis-à-vis de la protéine E7 native, c'est à dire la production d'anticorps qui reconnaissent la protéine E7 native et qui bloquent l'activité immunosuppressive de le protéine E7 native, délétère pour les cellules du système immunitaire. Selon l'invention, un anticorps anti-E7 est dit « neutralisant », notamment lorsqu'il bloque la capacité de la protéine E7 native à stimuler la production de TNF-$\alpha$ par des macrophages humains prétraités par de l'IFN-$\gamma$, selon le test décrit en détail à l'exemple 1.

Ces résultats ont permis de définir une famille de protéines E7 mutées qui ont toutes en commun de posséder des mutations, substitution(s) ou délétion d'acides aminés, par rapport à la séquence d'acides aminés de la protéine E7 native.

De manière générale, par protéine « E7 mutée » non immunosuppressive selon l'invention, on entend une protéine qui comprend la séquence en acides aminés qui consiste, de l'extrémité N-terminale, vers l'extrémité C-terminale, en :

- (i) la séquence d'acides aminés 1-19 de la séquence SEQ ID N°3 ;
- (ii) une séquence d'acides aminés possédant (a) la substitution d'au moins un, et de préférence d'au moins deux acides aminés, par rapport à la séquence correspondante d'acides aminés 20-29 de la séquence SEQ ID N°3 ou (b) la délétion d'au moins quatre acides aminés consécutifs, par rapport à la séquence correspondante d'acides aminés 20-29 de la séquence SEQ ID N°3 ; et
- (iii) la séquence d'acides aminés 30-98 de la séquence SEQ ID N°3.

Selon l'invention, une protéine E7 mutée qui « comprend » la séquence en acides aminés telle que définie ci-dessus est une protéine dont la séquence en acides aminés « consiste essentiellement » en la séquence d'acides aminés ci-dessus. En d'autres termes, une protéine E7 mutée selon l'invention peut comprendre, en plus de la séquence d'acides aminés ci-dessus, également une ou deux séquence(s) d'acides aminés additionnelle(s) d'au plus 20, avantageusement d'au plus 15, préférentiellement d'au plus 10, et de manière tout à fait préférée d'au plus 6 acides aminés,

la ou les séquence(s) d'acides aminés additionnelle(s) étant localisée(s) respectivement à l'extrémité N-terminale de la région (i) ou à l'extrémité C-terminale de la région (ii), et dans certains cas à la fois à l'extrémité N-terminale de la région (i) et à l'extrémité C-terminale de la région (ii). Dans le dernier cas, la séquence additionnelle localisée à l'extrémité N-terminale de la région (i) peut être distincte de la séquence additionnelle localisée à l'extrémité C-terminale, ou au contraire les deux séquences additionnelles peuvent être identiques. En général, une protéine E7 mutée selon l'invention comprend, par rapport à la séquence d'acides aminés ci-dessus qui la définit, une seule séquence d'acides aminés additionnelle, telle qu'une séquence d'acides aminés poly(histidine), par exemple une séquence consistant en un polymère de six résidus histidine.

Il résulte de la définition qui précède qu'une protéine E7 mutée selon l'invention possède deux régions d'acides aminés identiques aux régions peptidiques correspondantes de la séquence SEQ ID N°3, respectivement la région (i) d'acides aminés identique à la région peptidique 1-19 de la protéine E7 native de séquence SEQ ID N°3, et la région (iii) d'acides aminés identique à la région peptidique 30-98 de la protéine E7 native de séquence SEQ ID N°3. La région (ii) d'acides aminés centrale de la protéine E7 mutée comprend une ou plusieurs substitutions d'un acide aminé, ou alternativement comprend une délétion d'au moins quatre acides aminés, par rapport à la région peptidique 20-29 correspondante de la séquence SEQ ID N°3.

Selon la première alternative, la région (ii) d'acides aminés d'une protéine E7 mutée comprend de préférence au moins deux substitutions de nucléotides, et peut comprendre trois, quatre, cinq, six, sept, huit, neuf ou dix substitutions d'un acide aminé, par rapport aux acides aminés correspondants de la région peptidique 20-29 de la séquence SEQ ID N° 3 de la protéine native. Dans le mode de réalisation dans lequel la région (ii) d'acides aminés de la protéine E7 mutée comprend dix substitutions d'acides aminés, la région (ii) d'acides aminés de la protéine E7 mutée est totalement distincte de la région peptidique correspondante 20-29 de la protéine E7 native de séquence SEQ ID N°3.

Chaque substitution d'un acide aminé, dans la région 20-29 de la protéine E7 native, pour obtenir une protéine E7 mutée selon l'invention, est préférentiellement réalisée de manière à ce que l'acide aminé substitué dans la protéine E7 mutée appartienne à une « classe » distincte de l'acide aminé correspondant de la protéine E7 native, parmi les classes des acides aminés respectivement aromatiques, hydrophobes, polaires, basiques, acides, ou des petits acides aminés, avantageusement comme suit :

- résidu Thr en position 20 remplacé par un résidu autre que Ala, Ser, Met et Gly ;
- résidu Asp en position 21 remplacé par un résidu autre que Glu ;
- résidu Leu en position 22 remplacé par un résidu autre que Ile et Val ;
- résidu Tyr en position 23 remplacé par un résidu autre que Phe et Trp ;
- résidu Cys en position 24 remplacé par tout résidu d'acide aminé distinct ;
- résidu Tyr en position 25 remplacé par un résidu autre que Phe et Trp ;
- résidu Glu en position 26 remplacé par un résidu autre que Asp ;
- résidu Gln en position 27 remplacé par un résidu autre que Asn ;
- résidu Leu en position 28 remplacé par un résidu autre que Ile et Val ; et
- résidu Asn en position 29 remplacé par un résidu autre que Gln.

L'ensemble des acides aminés est constitué des acides aminés suivants : Ala, Arg, Asn, Asp, Cys, Gln, Glu, Gly, His, Ile, Leu, Lys, Met, Phe, Pro, Ser, Thr, Trp, Tyr et Val, ainsi que leurs dérivés chimiquement modifiés sur un ou plusieurs groupes chimiques ne participant pas à leur liaison avec un autre acide aminé dans la séquence d'une protéine E7 mutée selon l'invention.

Une famille préférée de protéines E7 mutées consiste en la famille de protéines dont les résidus Cys en position 24 et Glu en position 26 ont été substitués.

Une première protéine E7 mutée particulièrement préférée comprenant deux substitutions d'un nucléotide est la protéine E7 (CYS24GLY, GLU26GLN).

Une seconde protéine E7 mutée particulièrement préférée comprenant deux substitution d'un nucléotide est la protéine E7 (CYS24SER, GLU26GLN)

Selon la seconde alternative, la région (ii) d'acides aminés comprend la délétion de cinq, six, sept, huit, neuf ou dix acides aminés consécutifs, par rapport à la séquence correspondante d'acides aminés 20-29 de la séquence SEQ ID N°3. Lorsque la région (ii) d'acides aminés comprend la délétion de dix acides aminés, ladite région (ii) ne comprend aucun acide aminé et la protéine E7 mutée résultante consiste, de l'extrémité N-terminale vers l'extrémité C-terminale, en la région (i) 1-19 de la séquence SEQ ID N°3 qui est directement liée par une liaison peptidique à la région (iii) 30-98 de la séquence SEQ ID N°3.

Une première famille préférée de protéines E7 mutées selon la seconde alternative ci-dessus est la famille de protéines qui comprennent la délétion de six acides aminés consécutifs dans la région (ii), par rapport à la séquence correspondante d'acides aminés 20-29 de la séquence SEQ ID N°3.

Une troisième protéine E7 mutée préférée selon l'invention consiste en la protéine dont la séquence d'acides

aminés comprend la délétion des acides aminés 21 à 26 correspondants de la séquence SEQ ID N° 3 de la protéine E7 native. Cette protéine E7 mutée, aussi désignée E7Δ21-26, possède la séquence d'acides aminés SEQ ID N°1 et est codée par l'acide nucléique de séquence SEQ ID N°2. La région (ii) de la protéine E7Δ21-26 possède la délétion de six acides aminés consécutifs, par rapport à la séquence correspondante d'acides aminés 20-29 de la séquence SEQ ID N°3.

Une seconde famille préférée de protéines E7 mutées selon la seconde alternative ci-dessus est la famille de protéines qui comprennent la délétion de cinq acides aminés consécutifs dans la région (ii), par rapport à la séquence correspondante d'acides aminés 20-29 de la séquence SEQ ID N°3.

Une quatrième protéine E7 mutée préférée selon l'invention consiste en la protéine dont la séquence d'acides aminés comprend la délétion des acides aminés 21-25 correspondants de la séquence SEQ ID N° 3 de la protéine E7 native. Cette protéine E7 mutée, aussi désignée E7Δ21-25 dans la présente description. La région (ii) de la protéine E7Δ21-25 possède la délétion de cinq acides aminés consécutifs, par rapport à la séquence correspondante d'acides aminés 20-29 de la séquence SEQ ID N°3

Il est montré selon l'invention que le produit d'expression de l'ADN codant une protéine E7 mutée de HPV-16 telle que définie ci-dessus, lorsqu'il est combiné à un adjuvant de l'immunité approprié, est capable de générer chez la souris une réponse humorale et cellulaire spécifique, permettant ainsi d'induire une immunité anti-tumorale protectrice, comparable sinon supérieure, à celle induite par la protéine E7 native.

Par « produit d'expression » de l'ADN codant la protéine E7 mutée, on entend selon l'invention la ou les protéines résultant de la traduction de l'ADN ( ou de l'ARN messager correspondant) codant la protéine E7 mutée définie ci-dessus, dans une cellule hôte.

De plus, il est montré que le produit d'expression de l'ADN codant la protéine E7 mutée de HPV-16 selon l'invention possède une activité thérapeutique anti-tumorale égale ou supérieure à celle de la protéine E7 native.

En outre, un caractère essentiel du produit d'expression de l'ADN codant la protéine E7 mutée de l'invention, consiste, d'une part, en l'absence d'activité immunosuppressive directe de ce dernier et, d'autre part, en l'induction d'un blocage de l'immunosuppression induite par la protéine E7 native secrétée par les cellules cancéreuses.

La présente invention a pour objet une composition immunogène induisant une réponse immunitaire à l'encontre de la protéine E7 native du Papillomavirus HPV-16, sans induire simultanément une immunosuppression, comprenant, à titre de principe actif, une protéine E7 mutée non immunosuppressive telle que définie ci-dessus dans la présente description, le cas échéant en association avec un ou plusieurs excipients ou adjuvants de l'immunité physiologiquement compatible.

L'invention est aussi relative à une composition vaccinale dépourvue de propriété immunosuppressive, préventive ou curative à l'égard d'un cancer provoqué par une infection à *Papillomavirus*, **caractérisée en ce qu**'elle comprend, à titre de principe actif, une protéine E7 mutée non immunosuppressive telle que définie ci-dessus dans la présente description, en association avec un ou plusieurs adjuvants de l'immunité physiologiquement compatibles.

La présente invention concerne aussi l'utilisation d'une protéine E7 mutée non immunosuppressive telle que définie ci-dessus, pour la fabrication d'une composition immunogène ou d'une composition vaccinale non immunosuppressive et qui induit la production d'anticorps neutralisant l'activité immunosuppressive de la protéine E7 native.

Comme cela est illustré dans les exemples, une composition immunogène ou une composition vaccinale selon l'invention induit une réponse immunitaire cellulaire cytotoxique à l'encontre des cellules qui expriment la protéine E7 native, ou un peptide dérivé de la protéine E7 native. En particulier, il est montré dans les exemples qu'une composition immunogène ou une composition vaccinale selon l'invention induit une réponse immunitaire cellulaire cytotoxique à l'encontre de cellules tumorales exprimant la protéine E7 native, ou un peptide dérivé de la protéine E7 native.

La séquence en acides aminés de référence de la protéine E7 native de HPV-16 est, par exemple, la séquence décrite par Seedorf et al. (1985) dont le numéro d'accès dans la base de données Swissprot est N°P03129, et qui est reproduite dans la présente description comme la séquence SEQ ID N°3. L'acide nucléique codant la protéine E7 native de HPV-16 comprend la séquence nucléotidique SEQ ID N°4.

Une protéine E7 mutée telle que définie dans la présente description peut être préparée par toute technique conventionnelle de synthèse d'une protéine, bien connue de l'homme du métier.

Par exemple, une protéine E7 mutée peut être préparée par la technologie de l'ADN recombinant, par exemple par surexpression de l'acide nucléique codant celle-ci dans un système de cellules bactériennes recombinées, telles que des cellules de *E. coli* recombinées, comme décrit par Kiefer et al. (1996) et Tucker et al. (1996).

Une protéine E7 mutée peut aussi être préparée par voie de synthèse chimique, soit en solution homogène, soit en phase solide. Une première illustration d'une technique appropriée de synthèse chimique est par exemple celle décrite par Houben Weyl (1974).

Egalement, une protéine E7 mutée peut être préparée par une technique de synthèse chimique en solution ou en phase solide par des couplages successifs des différents résidus d'acides aminés qui doivent être incorporés (à partir de l'extrémité N-terminale jusqu'à l'extrémité C-terminale en phase liquide, ou de l'extrémité C-terminale jusqu'à l'extrémité N-terminale en phase solide), technique de synthèse dans laquelle les extrémités N-terminales et les chaînes

latérales des résidus d'acides aminés sont préalablement bloqués par des groupes chimiques protecteurs appropriés. Une illustration d'une technique qui peut être utilisée pour synthétiser une protéine E7 mutée est celle décrite par Merrifield (1965a ; 1965b).

## Acides nucléiques utilisés selon l'invention

Selon l'invention, une protéine E7 mutée peut être produite par traduction d'un acide nucléique codant ladite protéine E7 mutée, par exemple par un acide nucléique codant la protéine E7 mutée de séquence SEQ ID N°1 .

De préférence, tout acide nucléique et tout polypeptide selon l'invention se présentent sous une forme isolée ou purifiée.

Le terme « purifié » ne nécessite pas que le matériel soit présent sous une forme de pureté absolue, exclusive de la présence d'autres composés. Il s'agit plutôt d'une définition relative. Un polynucléotide ou un polypeptide est à l'état purifié après une purification du matériel de départ ou du matériel naturel d'au moins un ordre de grandeur, de préférence 2 ou 3 et préférentiellement 4 ou 5 ordres de grandeur.

Les termes « acide nucléique », « polynucléotide », « oligonucléotide » ou encore « séquence nucléotidique » englobe des séquences d'ARN, d'ADN, d'ADNc ou encore des séquences hybrides ARN/ADN d'au moins deux nucléotides, indifféremment sous la forme simple brin ou sous la forme de duplex.

Le terme « nucléotide » désigne à la fois les nucléotides naturels (A, T, G, C) ainsi que des nucléotides modifiés qui comprennent au moins une modification telle que (i) un analogue d'une purine, (ii) un analogue d'une pyrimidine, ou (iii) un sucre analogue, de tels nucléotides modifiés étant décrits par exemple dans la demande PCT N° WO 95/0 4064.

Aux fins de la présente invention, un premier nucléotide est considéré comme étant « complémentaire » d'un second polynucléotide lorsque chaque base du premier nucléotide est appariée à la base complémentaire du second polynucléotide dont l'orientation est inversée. Les bases complémentaires sont A et T ( ou A et U), et C et G.

Selon un mode de réalisation préféré, l'acide nucléique codant la protéine E7 mutée de séquence SEQ ID N°1 comprend la séquence nucléotidique SEQ ID N°2.

Un acide nucléique tel que défini ci-dessus est utile principalement lorsqu'il est mis en oeuvre pour la production du produit d'expression correspondant.

En conséquence, l'acide nucléique codant la protéine E7 mutée comprend, de préférence, un polynucléotide régulateur contrôlant l'expression de la protéine E7 mutée dans une cellule hôte procaryote ou eucaryote.

Le polynucléotide régulateur comprend au moins une séquence promoteur capable d'initier la transcription de l'ADN codant une protéine E7 mutée dans la cellule hôte choisie. Ce polynucléotide régulateur peut aussi comprendre d'autres séquences nucléotidiques favorisant l'expression de l'ADN codant la protéine E7 mutée, par exemple des séquences activatrices de la transcription bien connues de l'homme du métier.

Préférentiellement, on choisira un promoteur dit « inductible », c'est-à-dire un promoteur sensible à la présence d'un composé inducteur, ledit promoteur dirigeant la transcription de l'ADN placé sous son contrôle uniquement en présence du composé inducteur.

Le promoteur LacZ est un exemple illustratif d'un tel promoteur inductible.

Selon l'invention, toute technique classique de biologie moléculaire, de microbiologie et d'ADN recombinant connue de l'homme du métier peut être utilisée afin de préparer un acide nucléique tel que défini ci-dessus. De telles techniques sont décrites par exemple par Sambrook et al. (1989). Glover (1985), Gait (1984) et Ausubel et al. (1989).

L'acide nucléique comprenant un ADN codant une protéine E7 mutée selon l'invention, placé sous le contrôle d'un polynucléotide régulateur tel que défini ci-dessus est inclus dans une cassette d'expression.

## Cassettes d'expression

Une telle cassette d'expression peut comprendre, outre le polynucléotide régulateur de l'ADN codant la protéine E7 mutée, des séquences non traduites localisées du côté 5' du cadre ouvert de lecture, dites séquences « leader », capables d'augmenter la traduction du produit d'expression, ou des séquences dites « terminateur » bien connues de l'homme du métier.

De manière tout à fait préférée, une cassette d'expression telle que définie ci-dessus comprend en outre une séquence codant pour un peptide signal en vue de la production d'un polypeptide de fusion entre ledit peptide signal et la protéine E7, mutée, par exemple entre ledit peptide signal et la protéine E7Δ 21-26, afin de favoriser la sécrétion du produit d'expression de l'ADN codant la protéine E7 mutée et retrouver celui-ci principalement à l'extérieur des cellules, par exemple dans le surnageant de culture cellulaire. De préférence, la séquence nucléotidique codant le peptide signal est localisée immédiatement du côté 5' de la séquence nucléotidique codant la protéine E7 mutée.

En général, une cassette d'expression telle que définie ci-dessus comprend en outre un polynucléotide marqueur de sélection, par exemple le gène *his,* afin de sélectionner positivement les cellules hôtes transformées par celle-ci.

## Vecteurs recombinants

L'acide nucléique codant une protéine E7 mutée telle que définie dans la présente description, le cas échéant après insertion dans une cassette d'expression, est préférentiellement introduit dans un vecteur recombinant de clonage et/ou d'expression, qui comprend ledit acide nucléique ou ladite cassette d'expression, tels que définis ci-dessus.
Par « vecteur » au sens de la présente invention, on entend une molécule d'ADN ou d'ARN circulaire ou linéaire qui est indifféremment sous forme simple brin ou double brin.
Un vecteur recombinant est indifféremment un vecteur de clonage ou un vecteur d'expression.
Il peut s'agir d'un vecteur d'origine bactérienne ou virale.
Les vecteurs bactériens préférés selon l'invention sont par exemple les vecteurs Pbr322 (ATCC37017) ou encore des vecteurs tels que PAA223-3 (Pharmacia, Uppsala, Suède) et pGEM1 (Promega Biotech, Madison, WI, Etats-Unis).
On peut encore citer d'autres vecteurs commercialisés tels que les vecteurs pQE70, pQE60, pWE9 (Qiagen), psiX174, pBluescript SA, p NH8A, p NH16A, p NH18A, p NH46A, p WLNEO, p SV2CAT, p OG44, pXTI, pSG (Stratagene).
Selon un premier mode de réalisation, un vecteur recombinant tel que défini ci-dessus est utilisé afin d'amplifier l'acide nucléique ou la cassette d'expression qui y est insérée, après transformation ou transfection de l'hôte cellulaire désiré.
Selon un second mode de réalisation, il s'agit d'un vecteur d'expression comprenant un acide nucléique ou une cassette d'expression tels que définis ci-dessus, en vue de la transformation d'une cellule hôte choisie pour la production du produit d'expression de l'ADN codant une protéine E7 mutée selon l'invention.
Un vecteur recombinant tel que défini ci-dessus comprend avantageusement aussi des séquences d'initiation et d'arrêt de la transcription appropriée.
Une cassette d'expression telle que définie ci-dessus constitue un mode de réalisation particulier d'un vecteur recombinant selon l'invention.
Pour permettre l'expression de l'acide nucléique codant une protéine E7 mutée selon l'invention, un acide nucléique, une cassette d'expression ou un vecteur recombinant tel que défini ci-dessus doivent être introduits dans une cellule hôte.

## Cellules hôtes recombinantes

Pour mettre en oeuvre certains modes de production d'une protéine E7 mutée telle que définie dans la présente description, on a avantageusement recours à une cellule hôte transformée par un acide nucléique, une cassette d'expression ou un vecteur recombinant tels que définis ci-dessus.
La cellule hôte transformée est avantageusement une cellule bactérienne, telle qu'une cellule de *E. coli,* ou encore une cellule de levure, telle qu'une cellule de *Saccharomyces cerevisiae*.
Une telle cellule hôte transformée est avantageusement une cellule eucaryote.
Une catégorie de cellules hôtes recombinantes préférées sont les cellules de mammifères, telles que des cellules de souris, transformées par un acide nucléique, une cassette d'expression ou encore un vecteur recombinant selon l'invention.

## Procédé d'obtention d'un acide nucléique codant la protéine E7Δ21-26.

L'invention décrit également un procédé pour la fabrication d'un acide nucléique codant une protéine E7 mutée telle que définie dans la présente description, ledit procédé comprenant les étapes suivantes :

(a) réaliser une mutagenèse dirigée de l'acide nucléique codant la protéine E7 native, par amplification dudit acide nucléique à l'aide respectivement d'une première amorce nucléotidique et d'une seconde amorce nucléotidique hybridant avec l'extrémité 3' de l'ADN codant la protéine E7 native.
(b) récupérer l'ADN amplifié.

La première amorce nucléotidique comprend, dans sa séquence, la suite de codons codant pour les mutations que l'on souhaite insérer dans la séquence d'acides aminés de la protéine E7 native de départ.
Ainsi, si l'on souhaite préparer une protéine E7 mutée dont la région (ii) comprend une ou plusieurs substitutions d'un acide aminé, la séquence de la première amorce est telle qu'elle contient les codons appropriés codant pour le ou les acides aminés qui sont remplacés.
Si, selon une autre alternative, on souhaite préparer une protéine E7 mutée dont la région (ii) comprend la délétion de quatre, cinq, six, et, huit, neuf ou dix acides aminés consécutifs, par rapport à la séquence correspondante d'acides aminés 20-29 de la séquence SEQ ID N°3, alors la première amorce comprend, dans sa séquence, un premier

codon codant pour l'acide aminé localisé immédiatement du côté N-terminal du fragment peptidique à déléter, lequel premier codon précède immédiatement un second codon, ledit second codon codant pour l'acide aminé localisé immédiatement du côté N-terminal du fragment peptidique à déléter.

L'utilisation de la première amorce permet l'obtention d'un produit final d'amplification dans lequel les codons désirés ont été modifiés, lequel produit final d'amplification code la protéine E7 mutée désirée.

La seconde amorce nucléotidique utilisée à l'étape (a) d'amplification peut comprendre, outre une séquence hybridant avec l'extrémité 3' de l'ADN codant la protéine E7 native, également un ou plusieurs sites de reconnaissance par des endonucléases.

L'acide nucléique codant la protéine E7 mutée est inséré dans un vecteur de clonage ou dans un vecteur d'expression, à un site d'insertion prédéterminé, par exemple par ligation dudit acide nucléique avec l'acide nucléique du vecteur, après avoir préalablement ouvert le vecteur hôte, par exemple par coupure nucléotidique à l'aide d'une endonucléase de restriction, de préférence au niveau d'un polysite de clonage.

L'invention a pour objet une composition comprenant la protéine E7 mutée, telle que définie dans la présente description, et préférentiellement une composition immunogène ou une composition vaccinale comprenant ladite protéine E7 mutée.

**Compositions comprenant la protéine E7 mutée non immunosuppressive de l'invention.**

L'invention a pour objet toute composition comprenant une protéine E7 mutée non immunosuppressive selon l'invention, telle que la protéine E7 mutée comprenant la séquence en acides aminés de séquence SEQ ID N°1.

L'invention a également pour objet toute composition comprenant le produit d'expression de l'ADN codant une protéine E7 mutée selon l'invention, telle que la protéine E7Δ21-26 de séquence SEQ ID N°2.

On a montré selon l'invention qu'une protéine E7 mutée telle que définie dans la présente description est dépourvue de propriété immunosuppressive, comme cela est illustré dans les exemples.

En particulier, on a montré que la protéine E7Δ21-26 n'induisait aucune immunosuppression laquelle est administrée à un mammifère, en association avec un adjuvant de l'immunité approprié.

On a aussi montré que les protéines E7 mutées, respectivement la protéine E7(CYS24GLY, GLU26GLN) et la protéine E7 (CYS24SER, GLU26GLN), n'induisaient aucune immunosuppression lorsqu'elles sont administrées à un mammifère, en association avec un adjuvant de l'immunité approprié.

Comme cela a déjà été précisé succinctement précédemment, le produit d'expression de l'ADN codant la protéine E7 mutée de séquence SEQ ID N°1 induit une réponse immunitaire à l'encontre de la protéine E7 native au moins du même ordre que la réponse immunitaire induite par la protéine E7 native elle-même, Et la protéine E7 mutée selon l'invention est dépourvue, sur les cellules immunitaires humaines, des propriétés immunosuppressives qui caractérisent notamment la protéine E7 native.

L'invention a donc encore pour objet une composition pharmaceutique préventive ou curative d'une infection par HPV-16, et plus spécifiquement d'un cancer induit par une infection par HPV-16, **caractérisée en ce qu**'elle comprend, à titre de principe actif, le produit d'expression de l'ADN codant la protéine E7 mutée telle que définie ci-dessus, le cas échéant en association avec un ou plusieurs excipients physiologiquement compatibles.

De préférence, ladite composition pharmaceutique se présente sous une forme adaptée à l'administration d'une quantité hebdomadaire allant de 10 à 1000 μg de la protéine E7 mutée, ou du produit d'expression de l'ADN codant celle-ci.

Il est montré selon l'invention que le produit d'expression de l'ADN codant la protéine E7 mutée de séquence SEQ ID N°1, lorsqu'il est administré en combinaison avec un adjuvant de l'immunité approprié, est capable de générer une réponse immunitaire humorale et cellulaires dirigée contre la protéine E7 native permettant ainsi d'induire une immunité préventive à l'encontre des tumeurs. De plus, l'immunité protectrice induisant un état de résistance anti-tumorale provoquée par l'administration du produit d'expression de l'ADN codant la protéine E7 mutée est égale ou supérieure à celle qui est obtenue après immunisation préalable avec la protéine E7 native.

Il a aussi été montré que le produit d'expression de l'ADN codant la protéine E7 mutée de séquence SEQ ID N°1 est capable d'induire une immunité anti-tumorale thérapeutique, lorsque cette protéine est administrée, en association avec un adjuvant de l'immunité appropriée, à un individu ayant déjà développé des tumeurs. La réponse immunitaire anti-tumorale thérapeutique induite par le produit d'expression de l'ADN codant la protéine E7 mutée est significativement supérieure à celle qui peut être obtenue après administration de la protéine E7 native.

De tels résultats sont illustrés à l'exemple 3 où il est montré, dans un modèle murin, que l'immunité thérapeutique anti-tumorale induite par le produit d'expression de l'ADN codant la protéine E7 mutée permet d'allonger la durée de vie moyenne des animaux de 1,5 fois, par rapport à la durée de vie moyenne des animaux chez lesquels on a administré la protéine E7 native.

Il a également été montré que l'immunité antitumorale préventive de l'immunité antitumorale thérapeutique induite par le produit d'expression de l'ADN codant la protéine E7 mutée était due à l'induction simultanée d'une réponse

immunitaire humorale et cellulaires spécifique.

Ainsi, les résultats des exemples montrent qu'une protéine E7 mutée telle que définie dans la présente description, est capable d'induire une immunité humorale systémique à l'encontre de la protéine E7 native. Il a en particulier été montré qu'un haut niveau d'anticorps anti-E7 d'isotype IgG était produit, principalement des anticorps d'isotypes IgG2b et IgG1.

De plus, l'administration d'une protéine E7 mutée selon l'invention à un individu, dans les conditions appropriées, permet aussi d'induire une immunité de type mucosal, comme en témoignent le haut niveau de réponse anticorps d'isotype IgA présenté à l'exemple 3.

De plus, selon une caractéristique essentielle de la protéine E7 mutée, cette protéine qui est elle-même dépourvue de l'activité immunosuppressive directe de la protéine E7 native, permet aussi de bloquer l'activité immunosuppressive de la protéine E7 soluble sécrétée par les cellules cancéreuses, du fait du caractère neutralisant des anticorps produits après immunisation avec la protéine E7 mutée, comme en témoignent les résultats de l'exemple 2.

Les résultats des exemples montrent également qu'une protéine E7 mutée selon l'invention permet la prolifération de cellules TCD4+ et TCD8+ reconnaissant spécifiquement la protéine E7 native, cette prolifération cellulaire étant accompagnée par une surproduction de la cytokine IFN-$\gamma$.

L'invention est également relative à une composition immunogène induisant une réponse immunitaire à l'encontre de la protéine E7 native du Papillomavirus HPV-16, sans induire simultanément une immunosuppression, ladite composition comprenant, à titre de principe actif, une protéine E7 mutée non immunosuppressive dont la séquence en acides aminés consiste, de l'extrémité N-terminale, vers l'extrémité C-terminale, en :

- (i) la séquence d'acides aminés 1-19 de la séquence SEQ ID N°3 ;
- (ii) une séquence d'acides aminés possédant (a) la substitution d'au moins un acide aminé, par rapport à la séquence correspondante d'acides aminés 20-29 de la séquence SEQ ID N°3 ou (b) la délétion d'au moins quatre acides aminés consécutifs, par rapport à la séquence correspondante d'acides aminés 20-29 de la séquence SEQ ID N°3 ; et
- (iii) la séquence d'acides aminés 30-98 de la séquence SEQ ID N°3,

en association avec un ou plusieurs excipients ou adjuvants de l'immunité physiologiquement compatibles.

Une telle composition immunogène peut être utilisée de manière préventive, dans l'objectif d'induire une immunité anti-tumorale protectrice vis-à-vis d'une infection par HPV-16.

Une telle composition immunogène peut également être utilisée à titre thérapeutique ou curatif d'une infection par HPV-16, en particulier d'un cancer provoqué par une infection par HPV-16.

L'invention concerne également l'utilisation d'une protéine E7 mutée telle que définie dans la présente description pour la fabrication d'une composition pharmaceutique, ou pour la fabrication d'une composition immunogène telle que décrite ci-dessus.

La présente invention a également pour objet un procédé pour la fabrication d'une composition pharmaceutique, ou d'une composition immunogène, destinée à induire une immunité protectrice préventive ou une immunité thérapeutique à l'encontre d'un cancer provoqué par une infection par HPV-16, tout en bloquant l'état d'immunosuppression induit par la protéine E7 native, **caractérisé en ce qu**'il comporte une étape au cours de laquelle une protéine E7 mutée telle que définie ci-dessus est combiné avec un ou plusieurs adjuvants de l'immunité physiologiquement compatibles.

L'invention est également relative à une composition vaccinale préventive ou curative d'un cancer provoqué par une infection à papillomavirus **caractérisée en ce qu'**elle comprend, à titre de principe actif, une protéine E7 mutée non immunosuppressive dont la séquence en acides aminés consiste, de l'extrémité N-terminale, vers l'extrémité C-terminale, en :

- (i) la séquence d'acides aminés 1-19 de la séquence SEQ ID N°3 ;
- (ii) une séquence d'acides aminés possédant (a) la substitution d'au moins un acide aminé, par rapport à la séquence correspondante d'acides aminés 20-29 de la séquence SEQ ID N°3 ou (b) la délétion d'au moins quatre acides aminés consécutifs, par rapport à la séquence correspondante d'acides aminés 20-29 de la séquence SEQ ID N°3 ; et
- (iii) la séquence d'acides aminés 30-98 de la séquence SEQ ID N°3,

en association avec un ou plusieurs adjuvants de l'immunité physiologiquement compatibles.

Afin de lutter contre l'immunosuppression locale au niveau des cellules cancéreuses infectées par HPV-16, qui est induite par la protéine E7 extracellulaire, puis de stimuler une réponse immunitaire efficace à l'encontre de ces cellules cancéreuses, qui présentent à leur surface membranaire la protéine E7 native, ou des peptides dérivés de la protéine E7 native après maturation intracellulaire, en association avec un antigène de classe I du MHC, il est important d'induire la production d'anticorps, notamment d'anticorps de l'isotype IgG, capables de neutraliser l'effet immunosuppressif de la protéine E7 soluble sécrétée par les cellules cancéreuses infectées par le virus HPV-16. De plus, il est essentiel d'induire simultanément la production de lymphocytes cytotoxiques (CTL) capables de reconnaître spécifiquement la protéine E7 native, ou un peptide qui en est dérivé, exposé à la surface membranaire des cellules cancéreuses, afin de détruire ces cellules. Pour atteindre ces objectifs, il est nécessaire d'induire une réponse immunitaire de type systémique.

De plus, les papillomavirus humains étant détectés principalement dans les cancers des muqueuses, en particulier les cancers ano-génitaux, y compris le cancer du col utérin chez la femme, il est important de compléter les effets préventifs ou thérapeutiques de l'induction d'une réponse immunitaire de type systémique par l'induction d'une réponse immunitaire de type mucosal, notamment en stimulant la production d'anticorps d'isotype IgA au niveau local, sur les muqueuses.

Selon les objectifs poursuivis, on utilise des adjuvants de l'immunité capable d'orienter la réponse vers une immunité systémique ou mucosale.

Parmi les adjuvants de l'immunité systémique, on utilise de préférence les adjuvants de type IFA (adjuvant incomplet de Freund), le phosphate de calcium ou l'hydroxyde d'alumine. On peut aussi utiliser l'adjuvant QuilA, commercialisé par Brenntay Biosector, Danemark. Parmi les adjuvants de l'immunité mucosale, on utilise de préférence des adjuvants comme la choleratoxine B (CTB) ou encore un mutant de la toxine LT (LT$\mu$), bien connus de l'homme du métier.

L'induction d'une réponse immunitaire de type systémique et/ou ou de type mucosal est aussi influencée par la voie d'administration de la composition vaccinale de l'invention.

Ainsi, l'administration de la composition vaccinale par voie parentérale, sous-cutanée ou intradermique favorisera l'induction d'une réponse immunitaire systémique, accompagnée le cas échéant également d'une réponse immunitaire mucosale.

L'administration de la composition vaccinale selon l'invention au niveau local, par exemple par instillation nasale ou encore par application locale à la surface des muqueuses, favorisera l'induction d'une réponse immunitaire de type mucosal.

La présente invention a également pour objet une méthode de traitement préventif ou curatif d'un cancer provoqué par une infection par HPV-16 **caractérisée en ce qu**'elle comporte une étape au cours de laquelle on administre aux patients une quantité immunologiquement efficace d'une composition vaccinale telle que définie dans la présente description.

De préférence, on administre à un patient, sous une forme adaptée à l'administration systémique et/ou mucosale, une composition vaccinale de l'invention en quantité suffisante pour être efficace sur le plan préventif ou thérapeutique, à un sujet ayant besoin d'un tel traitement. La dose administrée de la protéine E7 mutée, ou du produit d'expression de l'ADN codant la protéine E7 mutée, peut aller par exemple de 10 à 1000 $\mu$g par voie parentérale, une fois par semaine pendant deux mois, puis périodiquement en fonction du niveau de la réponse cellulaire ou humorale induite, par exemple tous les deux à six mois.

Selon un premier aspect, la composition vaccinale selon l'invention est **caractérisée en ce qu**'elle comprend au moins un adjuvant susceptible d'orienter préférentiellement la réponse immunitaire vers la production d'anticorps neutralisant l'activité immunosuppressive de la protéine E7 sauvage.

Selon un premier mode de réalisation particulier, ladite composition vaccinale est **caractérisée en ce qu**'elle comprend au moins un adjuvant susceptible d'orienter préférentiellement la réponse immunitaire vers la production d'anticorps d' isotype IgA.

Selon un second mode de réalisation préféré, ladite composition vaccinale est **caractérisée en ce qu'e**lle comprend au moins un adjuvant susceptible d'orienter préférentiellement la réponse immunitaire vers la production d'anticorps d'isotype IgG.

Selon encore un autre mode de réalisation préféré, une composition vaccinale selon l'invention est **caractérisée en ce qu**'elle comprend la combinaison (i) d'un adjuvant susceptible d'orienter préférentiellement la réponse immunitaire vers la production d'anticorps d'isotype IgA et (ii) d'un adjuvant susceptible d'orienter préférentiellement la réponse immunitaire vers la production d'anticorps d'isotype IgG.

De préférence, la composition vaccinale selon l'invention est **caractérisée en ce qu**'elle comprend au moins un adjuvant de l'immunité susceptible d'induire une réponse immunitaire à la fois humorale et cellulaire.

De préférence, on cherchera à obtenir l'induction d'une réponse cellulaire, caractérisée notamment par la prolifération de lymphocytes T exprimant l'antigène CD8 et reconnaissant spécifiquement la protéine E7 sauvage, telle que présentée à la surface des cellules cancéreuses en association avec les antigènes de classe I du MHC.

Selon un autre mode de réalisation particulier d'une composition vaccinale selon l'invention, cette composition vaccinale peut comprendre un ou plusieurs autres composés antigéniques ou immunogènes de HPV. Par exemple, dans une composition vaccinale selon l'invention, le produit d'expression de l'ADN codant la protéine E7 mutée peut être associé à une ou plusieurs protéines de capside de HPV-16, ou à des peptides obtenus à partir de ces protéines de capside, en particulier les protéines L1 et L2 de HPV-16 bien connues de l'homme du métier.

Selon encore un autre mode de réalisation particulier, la composition vaccinale selon l'invention peut comprendre un ou plusieurs composés antigéniques ou immunogènes capables d'induire une réponse immunitaire cellulaire

ou humorale à l'encontre de types de HPV distincts de HPV-16, préférentiellement de type de HPV connus pour provoquer des cancers, tels que HPV-18, 31, 33 et 51.

Dans encore un autre mode de réalisation particulier d'une préparation vaccinale selon l'invention, le produit d'expression de l'ADN codant la protéine E7 mutée peut être combiné avec un ou plusieurs composés antigéniques ou immunogènes capables d'induire la production d'anticorps à l'encontre de facteurs solubles à propriétés immunosuppressives ou angiogéniques tels que l'IFN$\alpha$, le TGF$\beta$, le TNF$\alpha$ ou encore le VEGF. De préférence, ces composés antigéniques ou immunogènes sont respectivement l'IFN$\alpha$, le TGF$\beta$, le TNF$\alpha$ ou le VEGF modifiés chimiquement de manière à détruire leurs propriétés immunosuppressives et/ou à les stabiliser. De telles modifications chimiques, par exemple par carboxyméthylation, sont bien connues de l'homme du métier. Elles sont notamment décrites par Frankel et al. (1988).

Selon un aspect préféré de ce mode de réalisation particulier d'une composition vaccinale selon l'invention, le produit d'expression de l'ADN codant la protéine E7 mutée et au moins un autre composé antigénique ou immunogène sont liés chimiquement pour former un composé super immunogène tel que décrit dans la demande de brevet français n°01 10.751 déposée le 10 Août 2001 au nom de la Société NEOVACS.

***Superimmunogènes composites***

Un tel superimmunogène composite comprend deux polypeptides immunogènes distincts physiquement liés l'un à l'autre, les deux polypeptides consistant respectivement en :

(a) un premier polypeptide immunogène induisant une réaction immunitaire cellulaire, ou une réaction immunitaire cellulaire et humorale, à l'encontre d'une structure antigénique pathogène cellulaire de HPV16 ;

(b) un second polypeptide immunogène induisant la production d'anticorps neutralisants ou bloquants à l'encontre d'une protéine circulante locale du stroma choisie parmi un facteur cytokinique ou un facteur de régulation cellulaire à propriétés immunotoxiques ou angiogéniques, ce facteur étant produit par les cellules cancéreuses ou les cellules stromales, y compris les lymphocytes T et les cellules présentant l'antigène (APC).

Le superimmunogène composite utilisé selon l'invention est dit « bifonctionnel » car les deux polypeptides (a) et (b), qui sont les deux parties essentielles qui le constituent, permettent l'induction simultanée d'une réaction immunitaire dirigée contre deux cibles distinctes, respectivement la structure antigénique pathogène et la protéine circulante locale du stroma. Toutefois, un superimmunogène composite bifonctionnel selon l'invention peut comprendre dans sa structure une pluralité de copies respectivement d'un polypeptide (a) et/ou d'un polypeptide (b).

Le polypeptide (a) et le polypeptide (b) constitutifs d'un composé superimmunogène composite de l'invention sont dits « physiquement liés » l'un à l'autre car ils sont dans tous les cas inclus tous les deux dans une même structure physique, molécule ou particule (microparticule ou nanoparticule), au sein de laquelle ils sont peu distants l'un de l'autre. Du fait qu'ils sont physiquement liés l'un à l'autre dans le superimmunogène composite, le polypeptide (a) et le polypeptide (b) sont présentés conjointement aux mêmes cellules immunocompétentes, aussi bien les macrophages que les lymphocytes T ou B.

Selon un premier mode de réalisation préféré d'un superimmunogène composite, les polypeptides (a) et (b) sont choisis respectivement parmi :

- **Polypeptide (a) :** les protéines L1 et L2 du papillomavirus HPV-16, détoxiquée ou stabilisée si cela est nécessaire, des fragments immunogènes de ces protéines ou encore une protéine immunogène qui en est dérivée (Le Buanec et al., 1999) ;
- **Polypeptide (b) :** une protéine E7 mutée selon l'invention, par exemple une protéine E7 mutée de séquence SEQ ID N°1.

Selon un second mode de réalisation préféré d'un superimmunogène composite, les polypeptides (a) et (b) sont choisis respectivement parmi : :

- **Polypeptide (a) :** une protéine E7 mutée selon l'invention, par exemple la protéine E7 mutée de séquence SEQ ID N°1,
- **Polypeptide (b) :** les protéines IFN$\alpha$, TGF$\beta$, TNF$\alpha$ et VEGF, détoxiquées ou stabilisées si cela est nécessaire, des fragments immunogènes de ces protéines ou une protéine immunogène qui en est dérivée.

Selon un mode de réalisation avantageux, l'invention concerne aussi une composition comprenant plusieurs superimmunogènes composites se distinguant par l'identité de la protéine E7 mutée (polypeptide (a) ou polypeptide (b), selon le type de superimmunogène composite).

Pour fabriquer un superimmunogène composite selon l'invention, on réalise un couplage du polypeptide (a) et du polypeptide (b) par voie chimique ou par recombinaison génétique.

Dans un mode de réalisation particulier du conjugué peptidique immunogène de l'invention, les polypeptides (a) et (b) sont liés directement entre eux de manière covalente, par exemple via une liaison peptidique-CO-NH-.

Cependant, afin d'introduire une certaine flexibilité dans la structure du conjugué peptidique immunogène, et notamment de permettre une mobilité dans l'espace des polypeptides (a) et (b), l'un par rapport à l'autre au sein du conjugué peptidique immunogène, on préfère un conjugué peptidique dans lequel les polypeptides (a) et (b) sont séparés l'un de l'autre, au sein dudit conjugué, par une chaîne espaceur.

Selon un premier mode de réalisation préféré d'un conjugué peptidique immunogène, les polypeptides (a) et (b) sont séparés l'un de l'autre, au sein dudit conjugué, par une chaîne espaceur choisie parmi le SMCC ou le SIAB, qui sont tous les deux des composés bifonctionnels.

Le composé SIAB, décrit par Hermanson G.T. (1996, Bioconjugate techniques, San Diego : Academic Press, pp 239-242), est le composé de formule (I) suivante :

(I)

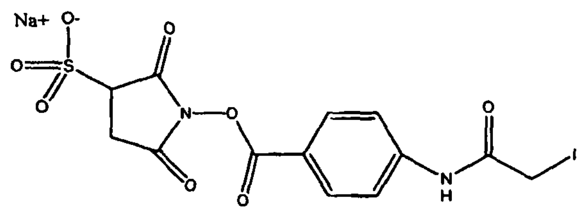

Le composé SIAB comprend deux groupes réactifs, respectivement un groupe iodoacétate et un groupe ester Sulfo-NHS, ces groupes réagissant respectivement sur des groupes amino et sulfhydryl.

Le composé SMCC, qui est décrit par Samoszuk M.K. et al. (1989, Antibody, Immunoconjugates Radiopharm., 2(1): 37-46), est le composé de formule (II) suivante :

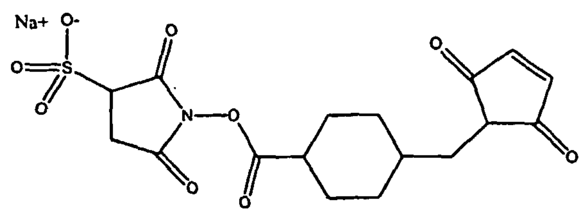

(II)

Le composé SMCC comprend deux groupes réactifs, respectivement un groupe ester Sulfo-NHS et un groupe maléimide, qui réagissent respectivement avec un groupe amino et un groupe sulfhydryl.

Selon un second mode de réalisation préféré, le superimmunogène composite comprend une chaîne espaceur constituée d'un peptide espaceur linéaire . On choisira de préférence un peptide espaceur linéaire ayant de 3 à 30 acides aminés de longueur, avantageusement de 5 à 20 acides aminés de longueur et de manière tout à fait préférée de 7 à 15 acides aminés de longueur.

Préférentiellement, le peptide espaceur linéaire est essentiellement, voire exclusivement, constitué d'acides aminés chargés positivement ou négativement à pH 7,0 afin d'accroître l'hydrophilicité globale dudit superimmunogène composite. On comprend que l'on devra éviter d'utiliser des peptides espaceurs constitués d'acides aminés hydrophobes. De manière préférentielle, le peptide espaceur est **caractérisé en ce qu**'il est constitué d'une chaîne de poly-(lysine ) constituée de 3 à 30 résidus lysine, avantageusement de 5 à 20, et de manière tout à fait préférée de 7 à 15 résidus lysine de longueur.

Selon encore un autre mode de réalisation d'un superimmunogène composite selon l'invention, les polypeptides (a) et (b) sont séparés l'un de l'autre, au sein dudit conjugué peptidique, par une chaîne espaceur constituée d'un peptide espaceur ramifié, de préférence une structure oligodendrimérique de poly(lysine), telle que décrite par exemple par

Basak et al. (1995).

Dans ce dernier mode de réalisation d'un superimmunogène composite selon l'invention, ledit conjugué peptidique peut comprendre plusieurs copies des polypeptides (a) et (b) par molécule de conjugué, avantageusement de 2 à 8 copies des polypeptides (a) et (b), de préférence au plus 4 copies de chacun des polypeptides (a) et (b) par molécule de conjugué.

Les polypeptides (a) et (b) peuvent aussi être inclus tous les deux au sein d'une même structure physique par exemple sur des nanoparticules ayant un diamètre compris entre 10 et 500 nanomètres, de préférence entre 10 et 2000 nanomètres, et de manière tout à fait préférée entre 10 et 100 nanomètres, par exemple des nanopaticules d'IMS, comme décrit par exemple par Aucouturier et al. (2001), de chitosan , comme décrit par exemple par Skaugrud et al. (1999), de liposomes, ou de particules biodégradables comme le polylactide acide (PLA), le poly-ε-caprolactone (PLC) ou le poly(lactide-coglycolide) (PLG) décrit par Baras et al. (1999).

Selon encore un autre mode de réalisation, les polypeptides (a) et (b) sont liés physiquement au sein d'une même structure porteuse permettant leur présentation simultanée aux cellules du système immunitaire. Dans ce mode de réalisation particulier, les polypeptides (a) et (b) sont immobilisés sur des nanoparticules, par exemple des nanoparticules de chitosan, de IMS (Immunomodulateur accessible auprès de la Société Seppic, France), constitué de nanoparticules lipidiques d'un diamètre de 100 à 300 nanomètres) ou de liposomes.

Préférentiellement, de telles nanoparticules sont de faible taille de manière à présenter simultanément les polypeptides (a) et (b) aux cellules, de la même manière que si ces polypeptides étaient liés de manière covalente au sein de la même molécule. Avantageusement les nanoparticules ont un diamètre compris entre 10 et 1000 nm, mieux entre 10 et 500 nm, préférentiellement entre 10 et 300 nm et de manière tout à fait préférée entre 10 et 200 nm.

**Compositions vaccinales contenant un acide nucléique codant la protéine E7 mutée selon l'invention.**

L'invention a aussi pour objet une composition immunogène, ou une composition vaccinale dépourvue de propriété immunosuppressive, préventive ou curative d'un cancer provoqué par une infection à papillomavirus, **caractérisée en ce qu**'elle comprend une quantité immunologiquement efficace d'un acide nucléique codant une protéine E7 mutée telle que définie dans la présente description , d'une cassette d'expression ou encore d'un vecteur recombinant tel que défini dans la présente description.

Pour la mise en oeuvre de la composition vaccinale ci-dessus, on préfère, en tant que vecteur d'expression, un vecteur rétroviral ou encore un vecteur adéno-associé (AAV). De tels vecteurs adéno-associés sont par exemple décrits par Flotte et al. (1992), Samulski et al. (1989), ou encore McLaughlin et al. (1996). Pour introduire les acides nucléiques, les cassettes d'expression ou les vecteurs dans une cellule hôte, l'homme du métier pourra avantageusement se référer à différentes techniques, comme la technique de précipitation au phosphate de calcium (Graham et al)., 1973, Chen et al. 1987), le DEAE dextran (Gopal, 1985), l'électroporation (Tur-kaspa, 1986, Potter et al. 1984), la microinjection directe (Harland et al. , 1985) ou encore les liposomes chargés en ADN (Nicolau et al., 1987 ; Fraley et al., 1980).

Selon un mode de réalisation particulier, une méthode pour introduire un acide nucléique ou une cassette d'expression selon l'invention dans une cellule hôte, en particulier une cellule hôte provenant d'un mammifère, *in vivo*, comprend une étape au cours de laquelle on introduit une préparation comprenant l'acide nucléique ou la cassette d'expression dans un véhicule pharmaceutiquement compatible, par injection locale au niveau du tissu choisi, par exemple un tissu musculaire lisse ou encore un tissu muqueux, l'acide nucléique ou la cassette d'expression étant absorbé par les cellules de ce tissu.

Des compositions pour l'utilisation *in vitro* et *in vivo* comprenant des acides nucléiques ou des cassettes d'expression « nus » sont par exemple décrites dans la demande PCT N°WO 95/11.307 ainsi que dans les articles de Tacson et al. (1996) et de Huygen et al. (1996).

Il peut encore s'agir de vecteurs adénoviraux tels que l'adénovirus humain de type 2 ou 5.

La quantité de vecteur qui est injectée à l'organisme hôte choisi varie selon le site de l'injection. A titre indicatif, il peut être injecté entre environ 10 μg et 1000 μg de l'acide nucléique ou de la cassette d'expression codant la protéine E7 mutée selon l'invention dans le corps d'un patient.

**Compositions destinées à la vaccination passive antitumorale.**

Comme cela apparaît clairement dans la présente description, la production d'anticorps systémiques ou mucosals est essentielle afin de bloquer les propriétés immunosuppressives de la protéine E7 sécrétée par les cellules cancéreuses infectées par HPV-16.

Dans la situation dans laquelle une composition vaccinale comprenant une protéine E7 mutée selon l'invention, ou la composition vaccinale comprenant un acide nucléique, une cassette d'expression ou un vecteur recombinant codant la protéine E7 mutée, est utilisée à titre thérapeutique , c'est-à-dire après l'événement d'infection par HPV-16, il est avantageux de bloquer rapidement l'effet immunosuppressif de la protéine E7 produite et sécrétée par les cellules

cancéreuses afin de favoriser l'induction rapide d'une réponse immunitaire humorale et cellulaire par ladite composition vaccinale, dans le but d'augmenter encore l'efficacité de celle-ci.

Le blocage précoce de l'effet immunosuppressif de la protéine E7 produite par les cellules infectées peut être réalisé en administrant aux patients une quantité efficace d'anticorps neutralisants obtenus après immunisation de l'homme ou de l'animal à l'encontre de la protéine E7 native, en utilisant une protéine E7 mutée de l'invention en tant que composé antigénique ou immunogène, en association avec un ou plusieurs adjuvants de l'immunité appropriée.

L'invention concerne aussi un procédé de préparation d'anticorps neutralisant l'effet immunosuppressif de la protéine E7 native de HPV-16, **caractérisé en ce qu**'il comporte une étape dans laquelle on immunise un mammifère, y compris l'homme, à l'aide d'une protéine E7 mutée de l'invention, puis on récupère les anticorps produits.

L'invention est aussi relative à des anticorps dirigés contre une protéine E7 mutée selon l'invention. Ces anticorps sont notamment **caractérisés en ce qu**'ils sont neutralisants, c'est-à-dire qu'ils neutralisent l'effet immunosuppressif de la protéine E7 native.

L'invention a également pour objet une composition pharmaceutique contenant des anticorps neutralisant l'effet immunosuppressif de la protéine E7 native de HPV 16, en particulier la protéine E7 sécrétée par des cellules infectées par HPV-16, lesdits anticorps étant dirigés contre une protéine E7 mutée selon l'invention.

Elle est aussi relative à une composition pour vaccination passive à l'encontre d'une infection par HPV-16, y compris à l'encontre d'un cancer provoqué par une infection par HPV-16, contenant les anticorps définis ci-dessus.

Pour vérifier la capacité neutralisante des anticorps selon l'invention, l'homme du métier se réfère avantageusement au test décrit à l'exemple 1 , consistant à quantifier le pourcentage d'inhibition de la production d'IFN-$\alpha$ ou de TNF-$\alpha$ par des macrophages humains exposés à la protéine E7 native.

L'invention a encore pour objet une méthode pour bloquer l'effet immunosuppressif de la protéine E7 produite par des cellules infectées par le virus HPV-16, **caractérisée en ce que** l'on administre aux patients une quantité neutralisante d'anticorps obtenus après immunisation de l'homme ou de l'animale avec une protéine E7 mutée telle que définie dans la présente description.

La « quantité neutralisante » d'anticorps à administrer au patient varie selon l'étendue de l'infection par HPV. En général, on administre au patient une quantité d'anticorps neutralisants qui correspond à la quantité d'anticorps neutralisants nécessaire à bloquer l'activité immunosuppressive d'une quantité de protéine E7 native allant de 100 ng à 1 mg, dans le test d'immunosuppression décrit à l'exemple 1..

Les anticorps peuvent être des anticorps d'isotype IgA ou d'isotype IgG. Ils peuvent être des anticorps polyclonaux ou encore des anticorps monoclonaux.

Les anticorps selon l'invention englobent également les fragments $F(ab')_2$ ou F(ab).

**Composition vaccinale comprenant des cellules dendritiques traitées avec une protéine E7 mutée selon l'invention.**

Les cellules dendritiques sont des cellules présentant l'antigène (APC) qui sont spécialisées dans l'initiation de l'immunité cellulaire T. Un contact physique entre des cellules dendritiques et des cellules T est requis pour l'induction d'une réponse immunitaire des cellules T. Les cellules dendritiques activent les réponses immunitaires spécifiques d'un antigène donné, via deux étapes de signalisation. La première étape de signalisation implique une interaction peptide antigénique-MHC/ récepteur des cellules T (TCR). La seconde étape de signalisation implique des molécules costimulatrices, tels que les marqueurs de surface cellulaires et les cytokines.

Les cellules dendritiques produisent diverses cytokines lorsqu'elles présentent l'antigène aux cellules T, ce qui influence le micro-environnement de cytokines, puis la réponse immunitaire subséquente.

Plus spécifiquement, on sait que la vaccination avec des cellules dendritiques permet de casser la tolérance immunologique du système immunitaire vis-à-vis des cellules tumorales et induit la lyse de la tumeur dans l'organisme hôte par la stimulation d'une réponse immunitaire cellulaire T de type Th1, tout au moins dans les cancers pour lesquels il n'existe pas de situation d'immunosuppression concomitante avec le développement de la tumeur.

On a montré selon l'invention que l'incubation *in vitro* d'une population de cellules dendritiques d'un individu avec une quantité appropriée d'une protéine E7 mutée, telle que définie dans la présente description, rendaient ces cellules dendritiques aptes à présenter subséquemment ladite protéine E7 mutée aux cellules T présentes dans une population de cellules mononucléées autologues, obtenues à partir du même individu, et à induire l'activation des cellules T spécifiques de la protéine E7, qui est visualisée par la prolifération de ces cellules T. L'activation des cellules T, peut être mesurée, par exemple, par la quantité d'incorporation intracellulaire de [$^3$H] thymidine, comme cela est illustré à l'exemple.

Dans une expérience similaire, mais dans laquelle les cellules dendritiques sont préalablement incubées *in vitro* avec la protéine E7 native, on n'observe aucune activation des cellules T, comme dans la population de cellules dendritiques témoin incubée en l'absence de tout antigène exogène. Dans ce système, on observe, une fois encore, l'activité immunosuppressive de la protéine E7 native.

Au contraire, une protéine E7 mutée selon l'invention est dépourvue d'activité immunosuppressive et permet l'induction d'une réponse immunitaire cellulaire T spécifique efficace, à l'encontre de la protéine E7 native.

L'invention a encore pour objet une composition vaccinale dépourvue de propriété immunosuppressive, préventive ou curative à l'égard d'un cancer provoqué par une infection à *Papillomavirus*, **caractérisée en ce qu**'elle comprend, à titre de principe actif, une quantité appropriée de cellules dendritiques autologues ou allogéniques vis-à-vis de l'individu à traiter, lesdites cellules dendritiques autologues ayant été incubées avec une protéine E7 mutée telle que définie dans la présente description et ainsi rendues aptes à présenter ladite protéine E7 mutée aux cellules T la reconnaissant spécifiquement.

L'invention a aussi pour objet une méthode pour prévenir ou guérir un cancer provoqué par une infection à *Papillomavirus*, **caractérisée en ce qu**'elle comprend une étape dans laquelle on administre à l'individu à traiter une quantité appropriée de cellules dendritiques autologues (isogéniques) ou allogéniques vis-à-vis de l'individu à traiter, lesdites cellules dendritiques autologues( isogéniques) ou allogéniques ayant été incubées, préalablement à leur administration à l'individu, avec une protéine E7 mutée telle que définie dans la présente description et ainsi rendues aptes à présenter ladite protéine E7 mutée aux cellules T la reconnaissant spécifiquement.

De préférence, les cellules dendritiques sont obtenues par différenciation de monocytes préparés par élutriation à partir de sang périphérique humain, soit du sang du patient, soit du sang d'un individu partageant avec le patient un haplotype du complexe majeut d'histocompatibilité, tel qu'un haplotype du HLA-A2.

De préférence, les cellules dendritiques, une fois prélevées à partir du patient, sont mise en culture dans un milieu de culture approprié pour la culture de cellules de mammifère, tels que le milieu HL1 (Bio Whittaker), le cas échéant additionné de glutamine, et éventuellement également d'un ou plusieurs antibiotiques, telle que la streptomycine et la culture est réalisée à 37°C dans un incubateur à atmosphère humide et à 5% (V/V) de $CO_2$..

Les cellules dendritiques en culture sont ensuite incubées avec une quantité choisie de la protéine E7 mutée, de préférence une quantité allant de 5 à 50 µg/ml.

Préférentiellement, on incube les cellules dendritiques avec la protéine E7 mutée, à raison d'une quantité de protéine E7 mutée par $10^6$ cellules dendritiques allant de 1 à 50 µg.

Préférentiellement, on administre au patient à traiter une quantité de cellules dendritiques traitées avec une protéine E7 mutée selon l'invention allant de $10^1$ à 500 x $10^6$ cellules.

Avantageusement, on réalise une seule administration au patient, par injection par la voie parentérale, des cellules dendritiques traitées par la protéine E7 mutée. On peut aussi réaliser, si nécessaire, de multiples injections au patient, en fonction de l'intensité de la réponse immunitaire anti-E7 qui est provoquée, par exemple une administration mensuelle, bimestrielle, trimestrielle ou bi-annuelle, des cellules dendritiques traitées

L'invention est en outre illustrée, sans pour autant être limitée, par les figures et les exemples suivants.

**Figure 1,** un graphe des titres d'anticorps IgG anti-E7 sérique de souris, avant et après immunisation avec une préparation E7Δ21-26 ;

**Figure 2,** un graphe du pourcentage de neutralisation des anticorps IgG anti-E7 sérique de souris, avant et après immunisation avec une préparation E7Δ21-26 ;

**Figure 3,** un graphe de l'index de prolifération Ip de splénocytes de souris naïves et de souris immunisées avec une préparation E7Δ21-26, cultivées en présence de la protéine E7 native ;

**Figure 4,** un graphe du taux d'IFN-γ de splénocytes de souris naïves et de souris immunisées avec une préparation E7Δ21-26, cultivées en présence de la protéine E7 native ;

**Figures 5 à 8,** des graphes du titre d'IgG anti-E7 sérique de souris et des pourcentages de neutralisation des anticorps IgG anti-E7 sérique de souris, avant et après immunisation avec une préparation E7Δ21-26 ;

**Figures 9 et 10,** des graphes du titre d'IgG anti-E7 sérique de souris et des pourcentages de neutralisation des anticorps IgG anti-E7 sériques de souris, avant et après immunisation avec une préparation E7Δ21-26 ;

**Figure 11,** un graphe représentant le taux d'IgA présent dans les sécrétions vaginales de souris, avant et après immunisation avec une préparation E7Δ21-26 ;

**Figures 12 et 13,** des graphes du titre d'IgG anti-E7 sérique de souris et des pourcentages de neutralisation des anticorps IgG anti-E7 sérique de souris, avant et après immunisation avec une préparation E7Δ21-26 ;

**Figure 14 ,** un graphe représentant le taux d'IgA présent dans les sécrétions vaginales de souris, avant et après immunisation avec une préparation E7Δ21-26 ;

**Figure 15** un graphe de la croissance tumorale de cellules C3 injectées à des souris immunisées ou non avec une préparation E7Δ21-26 .

**Figure 16** graphes représentant le développement des tumeurs C3 chez des souris ayant reçu 7 x $10^3$ cellules C3 exprimant E7 de HPV16. Figure **16A :** souris témoins ; Figure **16B :** souris traitées avec E7Δ21626. En abscisse, le nombre de jours après l'injection des cellules C3. En ordonnées, la surface des tumeurs.

Figure **17 :** graphes représentant le développement des tumeurs C3 chez des souris ayant reçu « 9 x $10^3$ » cellules C3 exprimant E7 de HPV16.

Figure **18 :** graphe représentant le niveau de réaction lymphocytaire mixte, mesurée par le niveau de prolifération des cellules mononucléées effectrices stimulées par les cellules dendritiques témoin (losange vide), prétraitées avec la protéine E7 native (cercle plein) ou prétraitées avec la protéine E7Δ21-26 (carré hachuré). En ordonnées prolifération des cellules mononucléées effectrices, visualisée par incorporation de [3H] thymidine. En abscisse, le rapport entre le nombre de cellules stimulantes (cellules dendritiques) et le nombre de cellules effectrices (cellules T).

**EXEMPLE 1 : Réponse immune (humorale et cellulaire) systémique.**

**Exemple 1.1: Activité immunogène de la protéine E7 Δ21-26 en présence d'adjuvant complet (ACF) et incomplet (AIF) de FREUND**

L'activité immunogénique (humorale et cellulaire) de la préparation E7 Δ21-26, a été étudiée chez les souris C57BU6 (H-$2^b$) femelles, âgées de 6 semaines, achetées chez Charles River, en présence de ACF et d'AIF.

***A. Matériel* et *méthodes***

Au jour 0, un groupe de 6 souris reçoit une injection de 0.2 ml (50 μg) d'une émulsion en ACF par voie intramusculaire. Une injection de rappel de 5 μg en AIF est donnée à J21 et J60.

Un prélèvement sanguin au niveau rétro-orbital est effectué sur chaque souris avant la première injection à j-2 et 12 jours après la dernière immunisation.

- 8 souris contrôles reçoivent les mêmes préparations sans immunogène.
- 3 souris reçoivent 100 μg de la préparation et l'absence de symptômes de maladie est étudiée pendant les 7 jours suivant l'injection.

Les souris sont challengées avec des cellules C3, 12 jours après la dernière immunisation. Les cellules C3, sont des cellules embryonnaires d'origine C57BU6, transformées avec le génome HPV16 entier et l'oncogène *ras* ( Feltkamp, M.C., H.L. Smits, M.P. Vierboom, R.P. Minnaar, B.M. de Jongh, J.W. Drijfhout, J. ter Schegget, C.J. Melief, and W.M. Kast. 1993. Vaccination with cytotoxic T lymphocyte epitope-containing peptide protects against a tumor induced by human papillomavirus type 16- transformed cells. Eur.J.Immunol. 23:2242-2249.). Elles sont cultivées dans du milieu DMEM (Bio-Whittaker) additionné de 10% de FCS, 50 U/ml de pénicilline, 50 μg/ml de streptomycine et 250 ng/ml de fungizone à 37°C dans une atmosphère humide contenant 7% de $CO_2$. Les cellules destinées à être injectées aux souris sont lavées dans du milieu sans sérum après avoir été détachées du plastique à l'aide de trypsine.

L'absence de toxicité de la préparation est mesurée par l'absence de signes cliniques : (comportement, poils, poids) et par examen anatomique après autopsie.

***B.* Résultats :**

Les souris immunisées par la préparation E7 Δ21-26, en présence de ACF et d'AIF, ne présentent aucun signe clinique et aucune lésion anatomique.

Aucune des 3 souris immunisées avec 100 μg de la préparation ne manifeste de symptômes de maladie pendant les 7 jours suivant l'injection.

La réaction immunitaire est mesurée par :

**1. Réponse humorale**

La présence dans le sérum d'anticorps de type IgG dirigés contre la protéine recombinante E7 native, est mesurée par ELISA et exprimée en titre (inverse de la dilution donnant une densité optique supérieure à 0.3)

**Figure 1 :**

Les souris immunisées avec la préparation de E7 Δ21-26, en présence de ACF et d'AIF, présentent des titres d'anticorps de type IgG anti-E7 très importants.

L'activité neutralisante de ces anticorps a été mesurée à l'aide du test d'immunosuppression suivant. Une dilution au 1/50 du sérum prélevé à J-2 et J72 sont incubées pendant 2 heures avec 50 ng/ml de E7 natif. Ces dilutions sont ensuite déposées sur des macrophages humains, prétraités par de l'IFN-γ pendant 16 heures. Après 24 heures de culture, les surnageants de culture sont récupérés et la quantité de TNF-α produite est mesurée par un test ELISA, le DuoSet DTA 50 (R&D). Les sérums neutralisants empêchent la protéine E7 d'induire l'expression de TNF-α, alors

que les sérums non neutralisants permettent la synthèse de cette cytokine.

Les résultats sont donnés en % de neutralisation.

**Figue 2**

Les anticorps induits par la préparation E7 Δ21-26 en présence d'ACF puis d'AIF ont un pouvoir neutralisant très important.

**2. Réponse cellulaire**

**2.1. Prolifération cellulaire**

Les cellules spléniques des souris immunisées et des souris contrôles sont isolées puis cultivées dans des puits à fond rond d'une plaque de micro-culture à raison de 100 000 cellules/puits en présence de E7 natif. La culture cellulaire est poursuivie à 37 ˚C en atmosphère humide chargée à 5 % de CO2 pendant 6 jours. 18 heures avant la fin de l'incubation, 0.5 μCi de thymidine tritiée/ puits sont ajoutés. L'intensité de la réponse immunitaire est proportionnelle à l'index de prolifération Ip.

Ip = cpm (coups par minute) pour l'antigène donné / cpm contrôle

**Figure 3**

Les cellules spléniques des souris immunisées avec la préparation E7 Δ21-26, en présence de ACF et d'AIF, prolifèrent, lorsqu'elles sont activées, in vitro, avec la protéine E7 native.

**2.2. Production d'IFN-γ**

La présence d'IFN gamma dans les surnageants de culture des cellules spléniques cultivées en présence de 10 μg/ml de E7 natif est déterminée, après 72 heures de culture, à l'aide d'un test ELISA (LO-IMEX, Belgium) comme décrit précédemment (De Smedt, T., B. Pajak, E. Muraille, L. Lespagnard, E. Heinen, P. De Baetselier, J. Urbain, O. Leo, and M. Moser. 1996. Regulation of dendritic cell numbers and maturation by lipopolysaccharide in vivo. J.Exp.Med. 184:1413-1424.) Les résultats sont exprimés en UI/ml.

**Figure 4**

Les cellules spléniques des souris immunisées avec la préparation E7 Δ21-26 en présence d'ACF puis d'AIF produisent une quantité importante d'IFN gamma lorsqu'elles sont activées, in vitro, avec la protéine E7 native.

**Exemple 1.2. : Activité immunogène de la protéine E7 Δ21-26 en présence d'adjuvant incomplet de FREUND (AIF)**

L'activité immunogénique (humorale) de la préparation E7 Δ21-26, a été étudiée chez les souris C57BL/6 (H-$2^b$) femelles, âgées de 6 semaines, achetées chez Charles River, en présence d'AIF.

***A. Matériel et méthodes***

Au jour 0, un groupe de 6 souris reçoit une injection de 0.2 ml (50 μg) d'une émulsion en AIF en présence de 1μg de GM-CSF murin et 1,5 μg d'IL-2 murin par voie intramusculaire. Une injection de rappel de 5 μg en AIF est donnée à J21 et J60.

Un prélèvement sanguin au niveau rétro-orbital est effectué sur chaque souris avant la première injection à j-2 et 12 jours après la dernière immunisation.

- 6 souris contrôles reçoivent les mêmes préparations sans immunogène.
- 3 souris reçoivent 100 μg de la préparation et l'absence de symptômes de maladie est étudiée pendant les 7 jours

suivant l'injection.

L'absence de toxicité de la préparation est mesurée par l'absence de signes cliniques : (comportement, poils, poids) et par examen anatomique après autopsie.

*B. Résultats :*

Les souris immunisées par la préparation E7 Δ21-26 en présence d'AIF ne présentent aucun signe clinique et aucune lésion anatomique.

Aucune des 3 souris immunisées avec 100 μg de la préparation ne manifeste de symptômes de maladie pendant les 7 jours suivant l'injection.

La réaction immunitaire est mesurée par :

**1. Réponse humorale**

La présence dans le sérum d'anticorps de type IgG dirigés contre la protéine recombinante E7 native, est mesurée par ELISA et exprimée en titre (inverse de la dilution donnant une densité optique supérieure à 0.3)

**Figure 5**

Les souris immunisées avec la préparation de E7 Δ21-26 en présence d'AIF présentent des titres d'anticorps de type IgG anti-E7 très importants.

L'activité neutralisante de ces anticorps a été mesurée à l'aide du test d'immunosuppression suivant. Une dilution au 1/50 du sérum prélevé à J-2 et J72 est incubée pendant 2 heures avec 50 ng/ml de E7 natif. Ces dilutions sont ensuite déposées sur des macrophages humains, prétraités par de l'IFN-γ pendant 16 heures. Après 24 heures de culture, les surnageants de culture sont récupérés et la quantité de TNF-α produite est mesurée par un test ELISA, le DuoSet DTA 50 (R&D). Les sérums neutralisants empêchent la protéine E7 d'induire l'expression de TNF-α, alors que les sérums non neutralisants permettent la synthèse de cette cytokine.

Les résultats sont donnés en % de neutralisation.

**Figure 6**

Les anticorps induits par la préparation E7 Δ21-26 en présence d'AIF ont un pouvoir neutralisant très important.

**Exemple 1.3 : Activité immunogène de la protéine E7 Δ21-26 enchassée dans des nanoparticules de chitosan en présence d'adjuvant incomplet de FREUND (AIF)**

L'activité immunogénique (humorale) de la préparation E7 Δ21-26 enchâssée dans des nanoparticules de chitosan a été étudiée chez les souris C57BU6 (H-$2^b$) femelles, âgées de 6 semaines, achetées chez Charles River, en présence d'AIF.

*A. Matériel et méthodes*

Au jour 0, un groupe de 6 souris reçoit une injection de 0.2 ml (50 μg) d'une émulsion en AIF en présence de 1μg de GMC-SF murin et 1,5 μg d'IL-2 murin par voie intramusculaire. Une injection de rappel de 5 μg en AIF est donnée à J21 et J60.

Un prélèvement sanguin au niveau rétro-orbital est effectué sur chaque souris avant la première injection à j-2 et 12 jours après la dernière immunisation.

- 6 souris contrôles reçoivent les mêmes préparations sans immunogène.
- 3 souris reçoivent 100 μg de la préparation et l'absence de symptômes de maladie est étudiée pendant les 7 jours suivant l'injection.

L'absence de toxicité de la préparation est mesurée par l'absence de signes cliniques : (comportement, poils, poids) et par examen anatomique après autopsie.

*B. Résultats :*

Les souris immunisées par la préparation E7 Δ21-26 enchâssée dans des nanoparticules de chitosan en présence d'AIF ne présentent aucun signe clinique et aucune lésion anatomique.
Aucune des 3 souris immunisées avec 100 μg de la préparation ne manifeste de symptômes de maladie pendant les 7 jours suivant l'injection.
La réaction immunitaire est mesurée par :

**1. Réponse humorale**

La présence dans le sérum d'anticorps de type IgG dirigés contre la protéine recombinante E7 native, est mesurée par ELISA et exprimée en titre (inverse de la dilution donnant une densité optique supérieure à 0.3)

**Figure 7**

Les souris immunisées avec la préparation de E7 Δ21-26 enchâssée dans des nanoparticules de chitosan en présence d'AIF présentent des titres d'anticorps de type IgG anti-E7 très importants.
L'activité neutralisante de ces anticorps a été mesurée à l'aide du test d'immunosuppression suivant. Une dilution au 1/50 du sérum prélevé à J-2 et J72 est incubée pendant 2 heures avec 50 ng/ml de E7 natif. Ces dilutions sont ensuite déposées sur des macrophages humains, prétraités par de l'IFN-γ pendant 16 heures. Après 24 heures de culture, les surnageants de culture sont récupérés et la quantité de TNF-α produite est mesurée par un test ELISA, le DuoSet DTA 50 (R&D). Les sérums neutralisants empêchent la protéine E7 d'induire l'expression de TNF-α, alors que les sérums non neutralisants permettent la synthèse de cette cytokine. Les résultats sont donnés en % de neutralisation.

**Figure 8**

Les anticorps induits par la préparation E7 Δ21-26 enchâssée dans des nanoparticules de chitosan en présence d'AIF ont un pouvoir neutralisant très important.

**EXEMPLE 2 : Réponse immunitaire systémique et mucosale**

**Exemple 2.1: Activité immunogène de la protéine E7 Δ21-26 en présence d'IMS 1113 (SEPPIC)**

L'activité immunogénique (humorale) de la préparation E7 Δ21-26, a été étudiée chez les souris C57BU6 (H-$2^b$) femelles, âgées de 6 semaines, achetées chez Charles River, en présence d'IMS 1113. (SEPPIC)

*A. Matériel et méthodes*

Au jour 0, un groupe de 6 souris reçoit une injection de 0.2 ml contenant 50 μg de E7 Δ21-26 en présence d'IMS 1113 avec 1μg de GM-CSF murin et 1,5 μg d'IL-2 murin par voie intramusculaire, ainsi que 20 μl contenant 20 μg de cette même préparation par voie intranasale.
Au jour 7, 14 et 21, ces souris reçoivent par voie intranasale 20 μl soit 20 μg de la préparation en IMS 1113.
Au jour 60, ces souris reçoivent une injection de 0.2 ml contenant 5 μg de la préparation en IMS 1113 et 20 μl contenant 20 μg de cette même préparation par voie intranasale.
Un prélèvement sanguin au niveau rétro-orbital ainsi qu'un prélèvement des sécrétions vaginales est effectué sur chaque souris avant la première injection à j-2 et 12 jours après la dernière immunisation.

- 6 souris contrôles reçoivent les mêmes préparations sans immunogène.
- 3 souris reçoivent 100 μg de la préparation et l'absence de symptômes de maladie est étudiée pendant les 7 jours suivant l'injection.

L'absence de toxicité de la préparation est mesurée par l'absence de signes cliniques : (comportement, poils, poids) et par examen anatomique après autopsie.

*B. Résultats :*

Les souris immunisées par la préparation E7 Δ21-26 en présence d'IMS 1113 ne présentent aucun signe

clinique et aucune lésion anatomique.

Aucune des 3 souris immunisées avec 100 μg de la préparation ne manifeste de symptômes de maladie pendant les 7 jours suivant l'injection.

La réaction immunitaire est mesurée par :

**1. Réponse humorale**

1.1. Production d'anticorps d'isotype G dans les sérums

La présence dans le sérum d'anticorps de type IgG dirigés contre la protéine recombinante E7 native, est mesurée par ELISA et exprimée en titre (inverse de la dilution donnant une densité optique supérieure à 0.3)

**Figure 9**

Les souris immunisées avec la préparation de E7 Δ21-26 en présence d'IMS 1113 présentent des titres d'anticorps de type IgG anti-E7 très importants.

L'activité neutralisante de ces anticorps a été mesurée à l'aide du test d'immunosuppression suivant. Une dilution au 1/50 du sérum prélevé à J-2 et J72 est incubée pendant 2 heures avec 50 ng/ml de E7 natif. Ces dilutions sont ensuite déposées sur des macrophages humains, prétraités par de l'IFN-γ pendant 16 heures. Après 24 heures de culture, les surnageants de culture sont récupérés et la quantité de TNF-α produite est mesurée par un test ELISA, le DuoSet DTA 50 (R&D). Les sérums neutralisants empêchent la protéine E7 d'induire l'expression de TNF-α, alors que les sérums non neutralisants permettent la synthèse de cette cytokine.

Les résultats sont donnés en % de neutralisation.

**Figure 10**

**1.2. Production d'anticorps d'isotype A dans les sécrétions vaginales**

La présence dans le sérum d'anticorps de type IgA dirigés contre la protéine recombinante E7 native, est mesurée par ELISA et exprimée en titre (inverse de la dilution donnant une densité optique supérieure à 0.3)

**Figure 11**

Les souris immunisées avec la préparation de E7 Δ21-26 en présence d'IMS 1113 présentent des titres d'anticorps de type IgA anti-E7 très importants.

**Exemple 2.2 : Activité immunogène de la protéine E7 Δ21-26 enchâssée dans du chitosan en présence d'AIF (voie systémique) ou en présence de PBS (voie nasale)**

L'activité immunogénique (humorale) de la préparation E7 Δ21-26 enchâssée dans du chitosan, a été étudiée chez les souris C57BL/6 ($H-2^b$) femelles, âgées de 6 semaines, achetées chez Charles River.

***A. Matériel et méthodes***

Au jour 0, un groupe de 6 souris reçoit une injection de 0.2 ml contenant 50 μg de E7 Δ21-26 enchâssée dans du chitosan en présence d'AIF avec 1μg de GM-CSF murin et 1,5 μg d'IL-2 murin par voie intramusculaire, ainsi que 20 μl contenant 20 μg de cette même préparation en présence de PBS additionnée d'1μg de GM-CSF murin et 1,5 μg d'IL-2 murin par voie intranasale.

Au jour 7, 14 et 21, ces souris reçoivent par voie intranasale 20 μl soit 20 μg de E7 Δ21-26 enchâssée dans du chitosan en présence de PBS.

Au jour 60, ces souris reçoivent une injection de 0.2 ml contenant 5 μg de la préparation en AIF et 20 μl contenant 20 μg de cette même préparation en présence de PBS par voie intranasale.

Un prélèvement sanguin au niveau rétro-orbital ainsi qu'un prélèvement des sécrétions vaginales est effectué sur chaque souris avant la première injection à j-2 et 12 jours après la dernière immunisation.

- 6 souris contrôles reçoivent les mêmes préparations sans immunogène.
- 3 souris reçoivent 100 μg de la préparation et l'absence de symptômes de maladie est étudiée pendant les 7 jours suivant l'injection.

L'absence de toxicité de la préparation est mesurée par l'absence de signes cliniques : (comportement, poils, poids) et par examen anatomique après autopsie.

**B.** ***Résultats*** *:*

Les souris immunisées par la préparation E7 Δ21-26 enchâssée dans du chitosan ne présentent aucun signe clinique et aucune lésion anatomique.

Aucune des 3 souris immunisées avec 100 μg de la préparation ne manifeste de symptômes de maladie pendant les 7 jours suivant l'injection.

La réaction immunitaire est mesurée par :

**1. Réponse humorale**

**1.1. Production d'anticorps d'isotype G dans les sérums**

La présence dans le sérum d'anticorps de type IgG dirigés contre la protéine recombinante E7 native, est mesurée par ELISA et exprimée en titre (inverse de la dilution donnant une densité optique supérieure à 0.3)

**Figure 12**

Les souris immunisées avec la préparation de E7 Δ21-26 enchâssée dans du chitosan présentent des titres d'anticorps de type IgG anti-E7 très importants.

L'activité neutralisante de ces anticorps a été mesurée à l'aide du test d'immunosuppression suivant. Une dilution au 1/50 du sérum prélevé à J-2 et J72 est incubée pendant 2 heures avec 50 ng/ml de E7 natif. Ces dilutions sont ensuite déposées sur des macrophages humains, prétraités par de l'IFN-γ pendant 16 heures. Après 24 heures de culture, les surnageants de culture sont récupérés et la quantité de TNF-α produite est mesurée par un test ELISA, le DuoSet DTA 50 (R&D). Les sérums neutralisants empêchent la protéine E7 d'induire l'expression de TNF-α, alors que les sérums non neutralisants permettent la synthèse de cette cytokine.

Les résultats sont donnés en % de neutralisation.

**Figure 13**

1.2. Production d'anticorps d'isotype A dans les sécrétions vaginales

La présence dans le sérum d'anticorps de type IgA dirigés contre la protéine recombinante E7 native, est mesurée par ELISA et exprimée en titre (inverse de la dilution donnant une densité optique supérieure à 0.3)

**Figure 14**

Les souris immunisées avec la préparation de E7 Δ21-26, enchâssée dans du chitosan, présentent des titres d'anticorps de type IgA anti-E7 très importants.

**EXEMPLE 3 : Réponse anti-tumorale protectrice**

**Exemple 3.1 : L'injection des protéines E7Δ21-26 en présence d'ACF puis AIF génère une résistance anti-tumorale.**

La mesure d'une réponse anti-tumorale protectrice chez les souris immunisées avec la préparation de E7 Δ21-26 en présence d'ACF puis d'AIF (exemple 1.1) a été testée de la manière suivante.

12 jours après la dernière immunisation, les souris ont été injectées avec 500.000 cellules C3, en sous-cutané (s-c) dans le flanc. Les cellules C3, sont des cellules embryonnaires d'origine C57BU6, transformées avec le génome HPV16 entier et l'oncogène ras. La croissance tumorale est évaluée 1 fois par semaine par la mesure de la surface de la tumeur.

**Figure 15**

Une tumeur s'est développée chez toutes les souris contrôles traitées uniquement avec l'adjuvant. Parmi les 6 souris immunisées avec la préparation E7 Δ21-26 en présence d'ACF puis d'AIF :

- 4 souris n'ont développé aucune tumeur
- chez 1 souris, une tumeur s'est développée puis stabilisée
- chez 1 souris, une tumeur s'est développée

La protéine E7 Δ21-26, génère chez la souris, en présence de ACF et d'AIF, une réponse immune spécifique protectrice contre une tumeur induite par le papillomavirus humain de type 16.

**EXEMPLE 4 : Réponse anti-tumorale thérapeutique**

La capacité de la préparation E7Δ21-26 en présence d'AIF à induire le rejet de tumeurs C3 préimplantées a été testée chez des souris C57 BU6(H-2b) de la manière décrite ci-dessous.

**Exemple 4.1 : Mise en place du modèle thérapeutique**

**A. Matériel et méthodes**

Des souris (groupes de 8) ont été injectées en s.c. dans le flanc avec un nombre croissant de cellules C3 : 0 ; $5.10^2$ ; $5.10^3$; $5.10^4$ et $5.10^5$. La croissance tumorale est évaluée 1 fois par semaine par la mesure de la surface de la tumeur.

**Résultats**

**TABLEAU I**

| Evaluation de la croissance tumorale par la mesure de la surface* des tumeurs | | | | | |
|---|---|---|---|---|---|
| **Nombre de cellules injectées** | **JO** | **J12** | **J17** | **J24** | **J31** |
| **Oe + 0** | **0** | **0** | **0** | **0** | **0** |
| **5e +2** | **0** | **0** | **0** | **0,1** | **1,9** |
| **5e + 3** | **0** | **0** | **0** | **0** | **2,8** |
| **5e + 4** | **0** | **0** | **0** | **10** | **6,9** |
| **5e + 5** | **0** | **25** | **202** | **sacrifiées** | **sacrifiées** |
| * La surface, exprimée en $mm^2$, représente la moyenne obtenue à partir de la mesure de surface de 8 tumeurs. | | | | | |

Les souris ayant reçu $5.10^5$ cellules ont toutes développé une tumeur 12 jours après l'injection de cellules (8/8). Parmi les souris ayant reçu $5.10^4$ cellules, 4 souris/8 ont développé une tumeur 21 jours après l'injection des cellules et 4 souris /8, 28 jours après l'injection.
Parmi les souris ayant reçu $5.10^3$ cellules, 1 souris /8 a développé une tumeur 35 jours après l'injection des cellules, 3 souris /8, 42 à
60 jours après l'injection et les 4 autres souris ne présentent toujours pas de tumeur.
Parmi les souris ayant reçu $5.10^2$ cellules, 1 souris /8 a développé une tumeur 35 jours après l'injection des cellules, les 7 autres souris n'ont toujours pas développé de tumeur.

**Exemple 4.2 : Réponse anti-tumorale chez la souris ayant reçu une dose de $7.10^3$ et $9.10^3$ cellules C3 exprimant la protéine E7**

**A. Matériel et méthodes**

Des souris ont d'abord été injectées en s.c. dans le flanc avec 7000 ou 9000 cellules C3 (jour 0). Le jour 2, 9, 16 et 23, les souris ont reçu une injection en intramusculaire de 10 μg de la préparation E7Δ21-26 dans de l'AIF et 1 μg de GM-CSF et d'IL2 murin. Les jours 9, 16, 23 et 60, les souris ont reçu une injection en intramusculaire de 10 μg de la préparation E7Δ21-26 dans de l'AIF.
La taille des tumeurs a été mesurée 2 fois par semaine comme décrit plus haut dans l'exemple 4.1.

**B. Résultats**

Les figures16 et 17 présentent les résultats obtenus.
Les souris contrôles développent toutes des tumeurs (8/8) 10 à 20 jours après l'injection des cellules C3 alors que 80 % (7 000 cellules injectées - Figure 16) et 75 % (9 000 cellules injectées - Figure 17) des souris immunisées n'en développent pas.

**Exemple 5 : Stimulation d'une réaction mixte lymphocytaire avec des cellules dendritiques prétraitées avec la protéine E7 mutée E7Δ21-26**

Une réaction lymphocytaire mixte autologue unidirectionnelle consiste à co-cultiver des cellules mononucléées d'un individu (cellules effectrices ou répondeuses) avec ses cellules dendritiques (cellules stimulantes) porteuses d'antigènes (TSA). Les cellules effectrices, possédant des récepteurs spécifiques pour l'antigène porté par les cellules stimulantes, prolifèrent. La prolifération est mesurée par le test d'incorporation à la thymidine tritiée.
Les cellules dendritiques proviennent de la différentiation de monocytes obtenus par élutriation à partir des PBMCs du sang périphérique d'un donneur (Sallusto et al, Journal of Experimental Medicine, 1994, 179, 1109-18). Des monocytes, à $2,5.10^6$ cellules/ml sont cultivés dans des poches de téflon dans du RPMI 1640 enrichi en SAB (10 %), en GMC-SF (50 ng/ml) et en IL4 (1 000 Unité /ml). Après 6 jours de culture, ces cellules présentent le phénotype de cellules dendritiques différentiées.
Ces cellules dendritiques ainsi obtenues, sont ensuite traitées avec une dose de 3 μg/ml de E7 natif, ou de E7 Δ21-26 et/ou du milieu de culture, puis cocultivées dans plaques 96 puits à fond rond avec les PBMCs du même donneur dans les rapports 1/10 ; 3/10 et 10/10. 18 heures avant la fin de la réaction, 0.5 μCi de thymidine tritiée/ puits sont ajoutés. Les résultats, donnés en coup par minute, sont présentés dans la Figure 18.
Lorsque les cellules dendritiques sont pré-incubées avec la protéine E7 native, puis cultivées avec les cellules répondantes, aucune prolifération n'est observée. (à cause de l'activité immunosupressive de la protéine E7 native). Lorsque les cellules dendritiques sont pré-incubées avec la protéine E7 mutée (dépourvue de l'activité immunosuppressive de la protéine native), une prolifération des stimulantes est observée.

**Exemple 6 :Absence d'activité immunossupressive des protéines E7 mutées.**

L'absence d'activité immunosuppressive in vitro a été mesurée à l'aide d'un test de prolifération cellulaire. Des cellules mononucléées du sang périphérique humain sont isolées puis cultivées dans des puits à fond rond à raison de 150 000 cellules/puits en présence de 3 μg/ml de différentes protéines E7 natives et de leur mutant respectif ou en présence de surnageant (SN) de culture de vaccine E7 HPV 16 (Δ21-25), don de MP Kieny, Transgène et en présence d'antigène de rappel (PPD + TT). La culture cellulaire est poursuivie à 37 °C en atmosphère humide chargée à 5 % de $CO_2$ pendant 6 jours. La prolifération cellulaire est mesurée par le test d'incorporation de thymidine tritiée. Les résultats, donnés en coups par minute sont résumés dans le Tableau 2 ci-dessous.

**Tableau 2**

| Contrôle milieu | 25 000 | 40 000 | 33 000 | 45 000 |
|---|---|---|---|---|
| PD1/3-HPV16-E7 | 5 000 | | | |
| PD1/3-HPV16-E7 mutant (C24G; E26Q) | 20 000 | | | |
| His-HPV16-E7 | | 10 000 | | |
| His-HPV16-E7 mutant (C24S; E26Q) | | 35 000 | | |
| His-HPV16-E7 | | | 7 500 | |
| His-HPV16-E7 mutant (Δ21-26) | | | 30 000 | |
| SN de vaccine E7 HPV 16 (Δ21-25) | | | | 40 000 |

Les résultats obtenus montrent que les mutants ont été détoxifiés (perte de l'activité immunosuppresive de la protéine native)

**REFERENCES**

- AUSUBEL F M, Brent R. Kingstone Re, Moore DD, Seidaman JG, Smith JA Struhl K editors , (1989), Current protocols in Molecular Biology, Wiley Interscience.
- AUCOUTURIER et al., 2001, Vaccine, 19 : 2666-2672.
- BARAS et al., 1999, Infect. Immun., 67: 2643-2648.
- BASAK et al., 1995, J. Pept. Sci., 1(6): 383-395.
- CHEN et al., 1987, Mol. Cell. Biol. Vol.7: 2745-2752.
- CRUZ , I. et al., 1999, Br. J. Cancer, vol.81 ; 881-889.
- De SMEDT, T., B. Pajak, E. Muraille, L. Lespagnard, E. Heinen, P. De Baetselier, J. Urbain, O. Leo, and M. Moser. 1996. Regulation of dendritic cell numbers and maturation by lipopolysaccharide in vivo. J.Exp.Med. 184:1413-1424.
- EL SHERIF AM et al., 2001, J. Pathol, 195(2): 179-185
- FRANKEL et al., 1988, Cell, vol.55.
- FELTKAMP, M.C., H.L. Smits, M.P. Vierboom, R.P. Minnaar, B.M. de Jongh, J.W. Drijfhout, J. ter Schegget, C.J. Melief, and W.M. Kast. 1993. Vaccination with cytotoxic T lymphocyte epitope-containing peptide protects against a tumor induced by human papillomavirus type 16-transformed cells. Eur.J.Immunol. 23:2242-2249.
- FRALEY 1980, J. Biol. Chem., vol.255:10431.
- FLOTTE et al., 1992, Am. J. Respir. cell Mol. Biol., vol. 7: 349-356
- GAIT (ed.), 1984, Nucleic acid hybridization.
- GERARD C M, 2001, Vaccine, vol. 19: 2583-2589.
- GIANNINI et al., 1998, Clin. EXP. Immunol, 113(2) : 183-189.
- GIANNINI ET AL., 2002, Int. J. CANCER , 97(5) : 654-659.
- GOPAL 1985, Mol. Cell. Biol., vol.5: 1188-1190.
- GLOVER (ed.), 1985, DNA Cloning: A practical approach, vol. I and II. Oligonuclotide synthesis, MRL Press, Ltd, Oxford, U.K.
- GRAHAM et al., 1973, Virology, vol.52: 456-457.
- HOUBEN WEYL, 1974, In Meuthode der Organischen Chemie, E. Wunsch Ed., Volume 15-I et 15-II, Thieme, Stuttgart.
- HUYGEN et al., 1996, Nature Medicine, vol.2 (8) : 893-898.
- HARLAND et al., 1985, J. Cell. Biol. vol.101 :1094-1095.
- KIEFER H. et al., 1996, Biochemistry, vol. 35(50):16077-16084.
- MCLAUGHLIN B A et al. , 1996, Am. J. Hum. Genet , vol.59:561-569.
- MERRIFIELD RB, 1965a, Nature, vol. 207(996):522-523.
- MERRIFIELD, 1965b, Science, vol. 150(693):178-185.
- MUDERSPACH L. et al., 2000, Clinical Cancer Research, vol.6: 3406-3416.
- N ICOLAU et al. , 1987, Methods enzymol., vol.149: 157-176.
- OSEN W et al., 2001, Vaccine, vol.19: 4276-4286.
- POTTER et al., 1984, Proc. Natl. Acad. sci. USA, vol.81 (22): 7161-7165.
- TUCKER J., GRISSHAMMER R., Biochem J., 1996, vol., 317(Pt3):891-899.
- SAMBROOK J FRITSCH E F et MANIATIS T, 1989, Molecular Cloning: A Laboratory Manual, 2 ed. Cold Spring Harbor Laboratory, Cold Spring Harbor, New York.
- SAMULSKI et al. 1989, J. Virol. , vol.63 :3822-3828.
- SCHOELL W M J, 1999, Gynecologic Oncology, vol.74: 448-455.
- SEEDORF K, 1985, Virology, vol. 145 (1): 181-185.
- SKAUGRUD et al. 1999, Biotechnol. Genet. Eng. Rev., 16: 23-40.
- TACSON et al., 1996, Nature Medicine, vol.2 (8) : 888-892.
- TUR-KASPA, 1986, Mol. Cell. Biol., vol.6: 716-718.


## Revendications

**1.** Composition vaccinale dépourvue de propriété immunosuppressive, préventive ou curative à l'égard d'un cancer provoqué par une infection à *Papillomavirus*, **caractérisée en ce qu'**elle comprend, à titre de principe actif, une protéine E7 mutée non immunosuppressive comprenant la séquence en acides aminés qui consiste, de l'extrémité N-terminale, vers l'extrémité C-terminale, en :

- (i) la séquence d'acides aminés 1-19 de la séquence SEQ ID N°3 ;
- (ii) une séquence d'acides aminés possédant (a) la substitution d'au moins un acide aminé, par rapport à la séquence correspondante d'acides aminés 20-29 de la séquence SEQ ID N°3 ou (b) la délétion d'au moins quatre acides aminés consécutifs, par rapport à la séquence correspondante d'acides aminés 20-29 de la séquence SEQ ID N°3 ; et
- (iii) la séquence d'acides aminés 30-98 de la séquence SEQ ID N°3,

en association avec un ou plusieurs adjuvants de l'immunité physiologiquement compatibles.

2. Composition vaccinale selon la revendication 1, dans laquelle la protéine E7 mutée est **caractérisée en ce que** la région (ii) d'acides aminés de ladite protéine E7 mutée comprend au moins deux substitutions d'acides aminés, par rapport aux acides aminés correspondants de la région peptidique 20-29 de la séquence SEQ ID N° 3 de la protéine native.

3. Composition vaccinale selon la revendication 2, dans laquelle la protéine E7 mutée est **caractérisée en ce que** la région (ii) d'acides aminés de ladite protéine E7 mutée comprend trois, quatre, cinq, six, sept, huit, neuf ou dix substitutions d'acides aminés, par rapport aux acides aminés correspondants de la région peptidique 20-29 de la séquence SEQ ID N° 3 de la protéine native.

4. Composition vaccinale selon la revendication 2, dans laquelle la protéine E7 mutée est **caractérisée en ce que** les résidus Cys en position 24 et Glu en position 26 ont été substitués par un résidu d'acide aminé distinct.

5. Composition vaccinale selon la revendication 4, dans laquelle la protéine E7 mutée est la protéine E7 **caractérisée par** CYS24GLY et GLU26GLN.

6. Composition vaccinale selon la revendication 4, dans laquelle la protéine E7 mutée est la protéine E7 **caractérisée par** CYS24SER et GLU26GLN.

7. Composition vaccinale selon la revendication 1 , dans laquelle la protéine E7 mutée est **caractérisée en ce que** la région (ii) d'acides aminés de ladite protéine E7 mutée comprend la délétion de cinq, six, sept, huit, neuf ou dix acides aminés consécutifs, par rapport à la séquence correspondante d'acides aminés 20-29 de la séquence SEQ ID N°3.

8. Composition vaccinale selon la revendication 7, dans laquelle la protéine E7 mutée est **caractérisée en ce que** la région (ii) d'acides aminés de ladite protéine E7 mutée comprend la délétion de six acides aminés consécutifs, par rapport à la séquence correspondante d'acides aminés 20-29 de la séquence SEQ ID N°3.

9. Composition selon la revendication 8, dans laquelle la protéine E7 mutée consiste en la protéine consiste en la protéine E7 mutée dont la séquence d'acides aminés comprend la délétion des acides aminés 21 à 26 correspondants de la séquence SEQ ID N° 3 de la protéine E7 native.

10. Composition vaccinale selon la revendication 7, dans laquelle la protéine E7 mutée est **caractérisée en ce que** la région (ii) d'acides aminés de ladite protéine E7 mutée comprend la délétion de cinq acides aminés consécutifs, par rapport à la séquence correspondante d'acides aminés 20-29 de la séquence SEQ ID N°3.

11. Composition vaccinale selon la revendication 10, dans laquelle la protéine E7 mutée consiste en la protéine consiste en la protéine E7 mutée dont la séquence d'acides aminés comprend la délétion des acides aminés 21 à 25 correspondants de la séquence SEQ ID N° 3 de la protéine E7 native.

12. Composition vaccinale selon l'une des revendications 1 à 11, **caractérisée en ce qu'**elle comprend au moins un adjuvant susceptible d'orienter préférentiellement la réponse immunitaire vers la production d'anticorps neutralisant l'activité immunosuppressive de la protéine E7 native.

13. Composition vaccinale selon la revendication 12, **caractérisée en ce qu'**elle comprend au moins un adjuvant susceptible d'orienter préférentiellement la réponse immunitaire vers la production d'anticorps d'isotype IgA.

14. Composition vaccinale selon la revendication 12, **caractérisée en ce qu'**elle comprend au moins un adjuvant susceptible d'orienter préférentiellement la réponse immunitaire vers la production, d'anticorps d'isotype IgG.

15. Composition vaccinale selon la revendication 12, **caractérisée en ce qu'**elle comprend la combinaison (i) d'un adjuvant susceptible d'orienter préférentiellement la réponse immunitaire vers la production d'anticorps d'isotype IgA et (ii) d'un adjuvant susceptible d'orienter préférentiellement la réponse immunitaire vers la production, d'anticorps d'isotype IgG.

16. Composition vaccinale selon la revendication 15, **caractérisée en ce qu'**elle comprend au moins un adjuvant de l'immunité susceptible d'induire une réponse immunitaire humorale et cellulaire.

**17.** Composition vaccinale selon la revendication 7, **caractérisée en ce que** la réponse cellulaire est **caractérisée** notamment par la prolifération de lymphocytes exprimant l'antigène CD8 et reconnaissant spécifiquemnt la protéine E7 sauvage.

**18.** Composition immunogène induisant une réponse immunitaire à l'encontre de la protéine E7 native du Papillomavirus HPV-16, sans induire simultanément une immunosuppression, ladite composition comprenant, à titre de principe actif, une protéine E7 mutée non immunosuppressive dont la séquence en acides aminés consiste, de l'extrémité N-terminale, vers l'extrémité C-terminale, en :

- (i) la séquence d'acides aminés 1-19 de la séquence SEQ ID N°3 ;
- (ii) une séquence d'acides aminés possédant (a) la substitution d'au moins un acide aminé, par rapport à la séquence correspondante d'acides aminés 20-29 de la séquence SEQ ID N°3 ou (b) la délétion d'au moins quatre acides aminés consécutifs, par rapport à la séquence correspondante d'acides aminés 20-29 de la séquence SEQ ID N°3 ; et
- (iii) la séquence d'acides aminés 30-98 de la séquence SEQ ID N°3,

en association avec un ou plusieurs excipients ou adjuvants de l'immunité physiologiquement compatibles.

**19.** Composition vaccinale préventive ou curative d'un cancer provoqué par une infection par HPV-16, **caractérisée en ce qu'**elle comprend une quantité immunologiquement efficace d'un acide nucléique codant une protéine E7 mutée telle que définie selon l'une des revendications 1 à 11, d'une cassette d'expression comprenant ledit acide nucléique ou d'un vecteur recombinant comprenant ladite cassette d'expression.

**20.** Utilisation d'une protéine E7 mutée non immunosuppressive telle que définie dans l'une quelconque des revendications 1 à 11, pour la fabrication d'une composition immunogène ou d'une composition vaccinale non immunosuppressive et qui induit la production d'anticorps neutralisant l'activité immunosuppressive de la protéine E7 native.

**21.** Anticorps neutralisants dirigés contre le produit d'expression de l'ADN codant la protéine E7 mutée telle que définie selon l'une des revendications 1 à 11.

**22.** Composition pour vaccination passive à l'encontre d'une infection par HPV-16 contenant des anticorps selon la revendication 21.

**23.** Composition vaccinale dépourvue de propriété immunosuppressive, préventive ou curative à l'égard d'un cancer provoqué par une infection à *Papillomavirus*, **caractérisée en ce qu'**elle comprend, à titre de principe actif, une quantité appropriée de cellules dendritiques autologues ou allogéniques vis-à-vis de l'individu à traiter, lesdites cellules dendritiques autologues ayant été incubées avec une protéine E7 mutée telle que définie dans l'une des revendications 1 à 11 et ainsi rendues aptes à présenter ladite protéine E7 mutée aux cellules T.

## Claims

**1.** A vaccine composition lacking preventive or curative immunosuppressive property towards a cancer caused by a Papillomavirus infection, **characterized in that** it comprises, as the active ingredient, a non immunosuppressive mutated E7 protein, comprising the amino acid sequence consisting, from the N-terminal end to the C-terminal end, of:

i. the 1-19 amino acid sequence of sequence SEQ ID No. 3;
ii. an amino acid sequence possessing (a) the substitution of at least one amino acid, compared to the 20-29 corresponding amino acid sequence of sequence SEQ ID No. 3 or (b) the deletion of at least four consecutive amino acids, compared to the 20-29 corresponding amino acid sequence of sequence SEQ ID No. 3; and
iii. the 30-98 amino acid sequence of sequence SEQ ID No. 3,in combination with one or more physiologically compatible immunity adjuvants.

**2.** A vaccine composition according to claim 1, wherein the mutated E7 protein is **characterized in that** the amino acid region (ii) of said mutated E7 protein comprises at least two substitutions of amino acids, compared to the corresponding 20-29 peptide region of amino acids of sequence SEQ ID No. 3 of the native protein.

**3.** A vaccine composition according to claim 2, wherein the mutated E7 protein is **characterized in that** the amino

acid region (ii) of said mutated E7 protein comprises three, four, five, six, seven, eight, nine or ten substitutions of amino acids, compared to the corresponding 20-29 peptide region of amino acids of sequence SEQ ID No. 3 of the native protein.

**4.** A vaccine composition according to claim 2, wherein the mutated E7 protein is **characterized in that** the Cys residues in position 24 and Glu in position 26 have been substituted by a distinct amino acid residue.

**5.** A vaccine composition according to claim 4, wherein the mutated E7 protein is the E7 protein **characterized by** CYS24GLY and GLU26GLN.

**6.** A vaccine composition according to claim 4, wherein the mutated E7 protein is the E7 protein **characterized by** CYS24SER and GLU26GLN.

**7.** A vaccine composition according to claim 1, wherein the mutated E7 protein is **characterized in that** the amino acid region (ii) of said mutated E7 protein comprises the deletion of five, six, seven, eight, nine or ten consecutive amino acids, compared to the corresponding 20-29 amino acid sequence of SEQ ID No. 3.

**8.** A vaccine composition according to claim 7, wherein the mutated E7 protein is **characterized in that** the amino acid region (ii) of said mutated E7 protein comprises the deletion of six consecutive amino acids, compared to the corresponding 20-29 amino acid sequence of SEQ ID No. 3.

**9.** A vaccine composition according to claim 8, wherein the mutated E7 protein consists of the mutated E7 protein having the amino acid sequence comprising the deletion of the corresponding amino acids 21 to 26 of the SEQ ID No. 3 sequence of the native E7 protein.

**10.** A vaccine composition according to claim 7, wherein the mutated E7 protein is **characterized in that** the amino acid region (ii) of said mutated E7 protein comprises the deletion of five consecutive amino acids, compared to the corresponding 20-29 amino acid sequence of SEQ ID No. 3.

**11.** A vaccine composition according to claim 10, wherein the mutated E7 protein consists of the mutated E7 protein having the amino acid sequence comprising the deletion of the corresponding amino acids 21 to 25 of the SEQ ID No. 3 sequence of the native E7 protein.

**12.** A vaccine composition according to any one of claims 1 to 11, **characterized in that** it comprises at least one adjuvant able to preferably direct the immune response towards the production of antibodies neutralizing the immunosuppressive activity of the native E7 protein.

**13.** A vaccine composition according to claim 12, **characterized in that** it comprises at least one adjuvant able to preferably direct the immune response towards the production of IgA isotype antibodies.

**14.** A vaccine composition according to claim 12, **characterized in that** it comprises at least one adjuvant able to preferably direct the immune response towards the production of IgG isotype antibodies.

**15.** A vaccine composition according to claim 12, **characterized in that** it comprises the combination (i) of one adjuvant able to preferably direct the immune response towards the production of IgA isotype antibodies and (ii) of one adjuvant able to preferably direct the immune response towards the production of IgG isotype antibodies.

**16.** A vaccine composition according to claim 15, **characterized in that** it comprises at least one immunity adjuvant able to induce a humoral and cellular immune response.

**17.** A vaccine composition according to claim 7, **characterized in that** the cellular response is **characterized** more particularly by the proliferation of lymphocytes expressing the CD8 antigen and specifically recognizing the wild-type E7 protein.

**18.** An immunogenic composition inducing an immune response towards the HPV-16 Papillomavirus native E7 protein, without simultaneously inducing an immunosuppression, said composition comprising, as the active ingredient, a non immunosuppressive mutated E7 protein, comprising the amino acid sequence consisting, from the N-terminal end to the C-terminal end, in:

i. the 1-19 amino acid sequence of sequence SEQ ID No. 3;
ii. an amino acid sequence possessing (a) the substitution of at least one amino acid, compared to the 20-29 corresponding amino acid sequence of sequence SEQ ID No. 3 or (b) the deletion of at least four consecutive amino acids, compared to the 20-29 corresponding amino acid sequence of sequence SEQ ID No. 3; and
iii. the 30-98 amino acid sequence of sequence SEQ ID No. ˚3, in combination with one or more physiologically compatible excipients or immunity adjuvants.

**19.** A preventive or curative vaccine composition for a cancer caused by a HPV-16 infection, **characterized in that** it comprises an immunologically effective amount of a nucleic acid encoding a mutated E7 protein, such as defined in any one of claims 1 to 11, of an expression cassette comprising the said nucleic acid or of a recombinant vector comprising the said expression cassette.

**20.** A use of a non immunosuppressive mutated E7 protein such as defined in any one of claims 1 to 11, for preparing a non immunosuppressive immunogenic composition or a non immunosuppressive vaccine composition and which induces the production of an antibodies neutralizing the immunosuppressive activity of the native E7 protein.

**21.** Neutralizing antibodies directed against the expression product of the DNA encoding the mutated E7 protein such as defined according to any one of claims 1 to 11.

**22.** A composition for passive vaccination towards a HPV-16 infection containing antibodies according to claim 21.

**23.** A vaccine composition lacking any preventive or curative immunosuppressive property towards a cancer caused by a Papillomavirus infection, **characterized in that** it comprises, as the active ingredient, an appropriate amount of dendritic cells that are autologous or allogenic as regard the individual to be treated, the said autologous dendritic cells having been incubated with a mutated E7 protein such as defined in any one of claims 1 to 11 and thereby made able to present the said mutated E7 protein to T cells.

**Patentansprüche**

**1.** Impfzusammensetzung ohne immunsuppressive Eigenschaft, die präventiv oder heilend auf eine Krebserkrankung wirkt, die durch eine Papillomvirus-Infektion hervorgerufen ist, **dadurch gekennzeichnet, dass** sie Folgendes umfasst: als Wirkstoff ein mutiertes, nicht immunsuppressives E7-Protein, das die Aminosäuresequenz umfasst, die vom N-terminalen Ende zum C-terminalen Ende aus Folgendem besteht:

- (i) der Sequenz der Aminosäuren 1-19 der Sequenz SEQ ID Nr. 3;
- (ii) einer Aminosäuresequenz, die (a) eine Substitution von wenigstens einer Aminosäure in Bezug auf die entsprechende Sequenz der Aminosäuren 20-29 der Sequenz SEQ ID Nr. 3 oder (b) eine Deletion von wenigstens vier aufeinanderfolgenden Aminosäuren in Bezug auf die entsprechende Sequenz der Aminosäuren 20-29 der Sequenz SEQ ID Nr. 3 besitzt; und
- (iii) der Sequenz der Aminosäuren 30-98 der Sequenz SEQ ID Nr. 3;

in Verbindung mit einem oder mehreren physiologisch verträglichen Adjuvantien der Immunität.

**2.** Impfzusammensetzung gemäß Anspruch 1, wobei das mutierte E7-Protein **dadurch gekennzeichnet ist, dass** der Aminosäurebereich (ii) des mutierten E7-Proteins wenigstens zwei Aminosäuresubstitutionen in Bezug auf die entsprechenden Aminosäuren des Peptidbereichs 20-29 der Sequenz SEQ ID Nr. 3 des nativen Proteins umfasst.

**3.** Impfzusammensetzung gemäß Anspruch 2, wobei das mutierte E7-Protein **dadurch gekennzeichnet ist, dass** der Aminosäurebereich (ii) des mutierten E7-Proteins drei, vier, fünf, sechs, sieben, acht, neun oder zehn Aminosäuresubstitutionen in Bezug auf die entsprechenden Aminosäuren des Peptidbereichs 20-29 der Sequenz SEQ ID Nr. 3 des nativen Proteins umfasst.

**4.** Impfzusammensetzung gemäß Anspruch 2, wobei das mutierte E7-Protein **dadurch gekennzeichnet ist, dass** die Reste Cys auf Position 24 und Glu auf Position 26 durch einen unterschiedlichen Aminosäurerest substituiert sind.

**5.** Impfzusammensetzung gemäß Anspruch 4, wobei es sich bei dem mutierten E7-Protein um das Protein E7 handelt, das durch CYS24GLY und GLU26GLN **gekennzeichnet** ist.

6. Impfzusammensetzung gemäß Anspruch 4, wobei es sich bei dem mutierten E7-Protein um das Protein E7 handelt, das durch CYS24SER und GLU26GLN **gekennzeichnet** ist.

7. Impfzusammensetzung gemäß Anspruch 1, wobei das mutierte E7-Protein **dadurch gekennzeichnet ist, dass** der Aminosäurebereich (ii) des mutierten E7-Proteins die Deletion von fünf, sechs, sieben, acht, neun oder zehn aufeinanderfolgenden Aminosäuren in Bezug auf die entsprechende Sequenz der Aminosäuren 20-29 der Sequenz SEQ ID Nr. 3 umfasst.

8. Impfzusammensetzung gemäß Anspruch 7, wobei das mutierte E7-Protein **dadurch gekennzeichnet ist, dass** der Aminosäurebereich (ii) des mutierten E7-Proteins die Deletion von sechs aufeinanderfolgenden Aminosäuren in Bezug auf die entsprechende Sequenz der Aminosäuren 20-29 der Sequenz SEQ ID Nr. 3 umfasst.

9. Zusammensetzung gemäß Anspruch 8, wobei das mutierte E7-Protein aus dem mutierten E7-Protein besteht, dessen Aminosäuresequenz die Deletion der entsprechenden Aminosäuren 21 bis 26 der Sequenz SEQ ID Nr. 3 des nativen E7-Proteins umfasst.

10. Impfzusammensetzung gemäß Anspruch 7, wobei das mutierte E7-Protein **dadurch gekennzeichnet ist, dass** der Aminosäurebereich (ii) des mutierten E7-Proteins die Deletion von fünf aufeinanderfolgenden Aminosäuren in Bezug auf die entsprechende Sequenz der Aminosäuren 20-29 der Sequenz SEQ ID Nr. 3 umfasst.

11. Impfzusammensetzung gemäß Anspruch 10, wobei das mutierte E7-Protein aus dem mutierten E7-Protein besteht, dessen Aminosäuresequenz die Deletion der entsprechenden Aminosäuren 21 bis 25 der Sequenz SEQ ID Nr. 3 des nativen E7-Proteins umfasst.

12. Impfzusammensetzung gemäß einem der Ansprüche 1 bis 11, **dadurch gekennzeichnet, dass** sie wenigstens ein Adjuvans umfasst, das die Immunantwort vorzugsweise auf die Produktion von Antikörpern ausrichten kann, die die immunsuppressive Aktivität des nativen E7-Proteins neutralisieren.

13. Impfzusammensetzung gemäß Anspruch 12, **dadurch gekennzeichnet, dass** sie wenigstens ein Adjuvans umfasst, das die Immunantwort vorzugsweise auf die Produktion von Antikörpern des Isotyps IgA ausrichten kann.

14. Impfzusammensetzung gemäß Anspruch 12, **dadurch gekennzeichnet, dass** sie wenigstens ein Adjuvans umfasst, das die Immunantwort vorzugsweise auf die Produktion von Antikörpern des Isotyps IgG ausrichten kann.

15. Impfzusammensetzung gemäß Anspruch 12, **dadurch gekennzeichnet, dass** sie eine Kombination von (i) einem Adjuvans, das die Immunantwort vorzugsweise auf die Produktion von Antikörpern des Isotyps IgA ausrichten kann, und (ii) einem Adjuvans, das die Immunantwort vorzugsweise auf die Produktion von Antikörpern des Isotyps IgG ausrichten kann, umfasst.

16. Impfzusammensetzung gemäß Anspruch 15, **dadurch gekennzeichnet, dass** sie wenigstens ein Adjuvans der Immunität umfasst, das eine humorale und zelluläre Immunantwort induzieren kann.

17. Impfzusammensetzung gemäß Anspruch 7, **dadurch gekennzeichnet, dass** die zelluläre Antwort insbesondere durch die Vermehrung von Lymphocyten, die das Antigen CD8 exprimieren und spezifisch das Wildtyp-E7-Protein erkennen, **gekennzeichnet** ist.

18. Immunogene Zusammensetzung, die eine Immunantwort gegen das native E7-Protein des Papillomvirus HPV-16 induziert, ohne gleichzeitig eine Immunsuppression zu induzieren, wobei die Zusammensetzung Folgendes umfasst: als Wirkstoff ein mutiertes, nicht immunsuppressives E7-Protein, das die Aminosäuresequenz umfasst, die vom N-terminalen Ende zum C-terminalen Ende aus Folgendem besteht:

    - (i) der Sequenz der Aminosäuren 1-19 der Sequenz SEQ ID Nr. 3;
    - (ii) einer Aminosäuresequenz, die (a) eine Substitution von wenigstens einer Aminosäure in Bezug auf die entsprechende Sequenz der Aminosäuren 20-29 der Sequenz SEQ ID Nr. 3 oder (b) eine Deletion von wenigstens vier aufeinanderfolgenden Aminosäuren in Bezug auf die entsprechende Sequenz der Aminosäuren 20-29 der Sequenz SEQ ID Nr. 3 besitzt; und
    - (iii) der Sequenz der Aminosäuren 30-98 der Sequenz SEQ ID Nr. 3;

in Verbindung mit einem oder mehreren physiologisch verträglichen Arzneimittelhilfsstoffen oder Adjuvantien der Immunität.

**19.** Impfzusammensetzung, die präventiv oder heilend auf eine Krebserkrankung wirkt, die durch eine Infektion durch HPV-16 hervorgerufen ist, **dadurch gekennzeichnet, dass** sie Folgendes umfasst: eine immunologisch wirksame Menge einer Nucleinsäure, die ein mutiertes E7-Protein codiert, wie es gemäß einem der Ansprüche 1 bis 11 definiert ist, einer Expressionscassette, die die Nucleinsäure umfasst, oder eines Vektors, der die Expressionscassette umfasst.

**20.** Verwendung eines nicht immunsuppressiven mutierten E7-Proteins, wie es in einem der Ansprüche 1 bis 11 definiert ist, zur Herstellung einer immunogenen Zusammensetzung oder einer nicht immunsuppressiven Impfzusammensetzung, die die Produktion von Antikörpern induziert, welche die immunsuppressive Aktivität des nativen E7-Proteins neutralisieren.

**21.** Neutralisierende Antikörper, die gegen das Expressionsprodukt der DNA gerichtet sind, die das mutierte E7-Protein, wie es gemäß einem der Ansprüche 1 bis 11 definiert ist, codiert.

**22.** Zusammensetzung zur passiven Impfung gegen eine Infektion durch HPV-16, enthaltend Antikörper gemäß Anspruch 21.

**23.** Impfzusammensetzung ohne immunsuppressive Eigenschaft, die präventiv oder heilend auf eine Krebserkrankung wirkt, die durch eine Papillomvirus-Infektion hervorgerufen ist, **dadurch gekennzeichnet, dass** sie Folgendes umfasst: als Wirkstoff eine geeignete Menge von Dendrocyten, die autolog oder allogen in Bezug auf das zu behandelnde Individuum sind, wobei die autologen Dendrocyten mit einem mutierten E7-Protein, wie es gemäß einem der Ansprüche 1 bis 11 definiert ist, inkubiert wurden und **dadurch** befähigt sind, das mutierte E7-Protein gegenüber T-Zellen zu präsentieren.

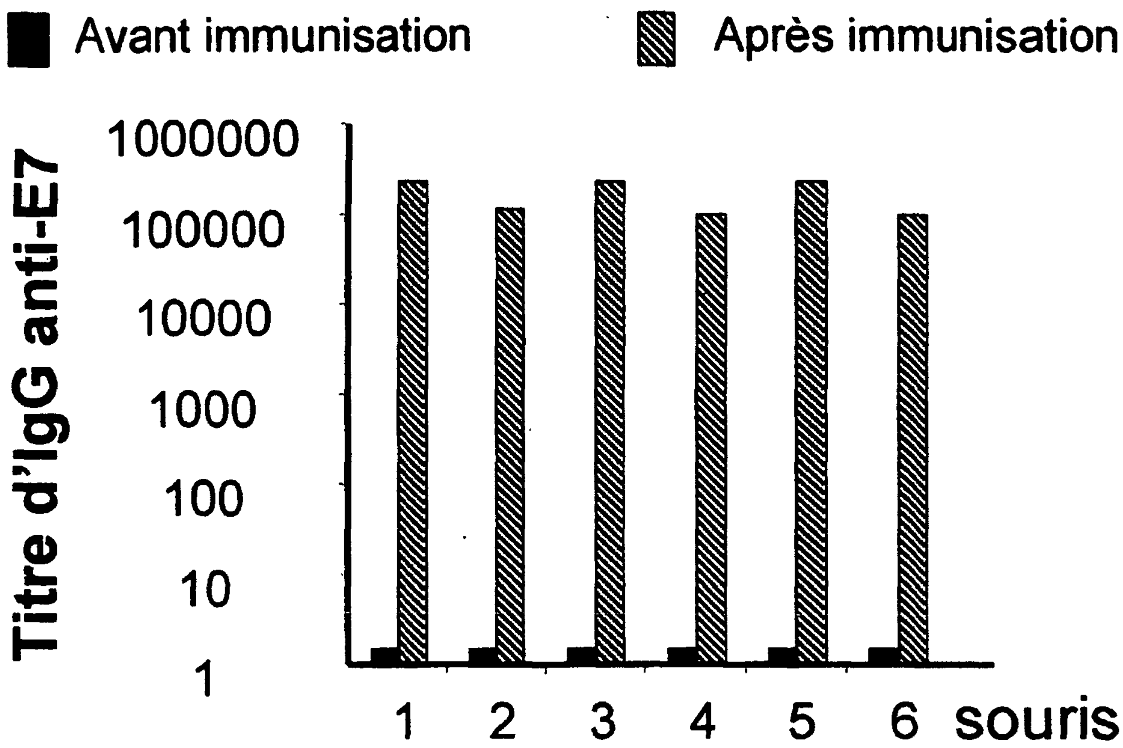


FIGURE 1

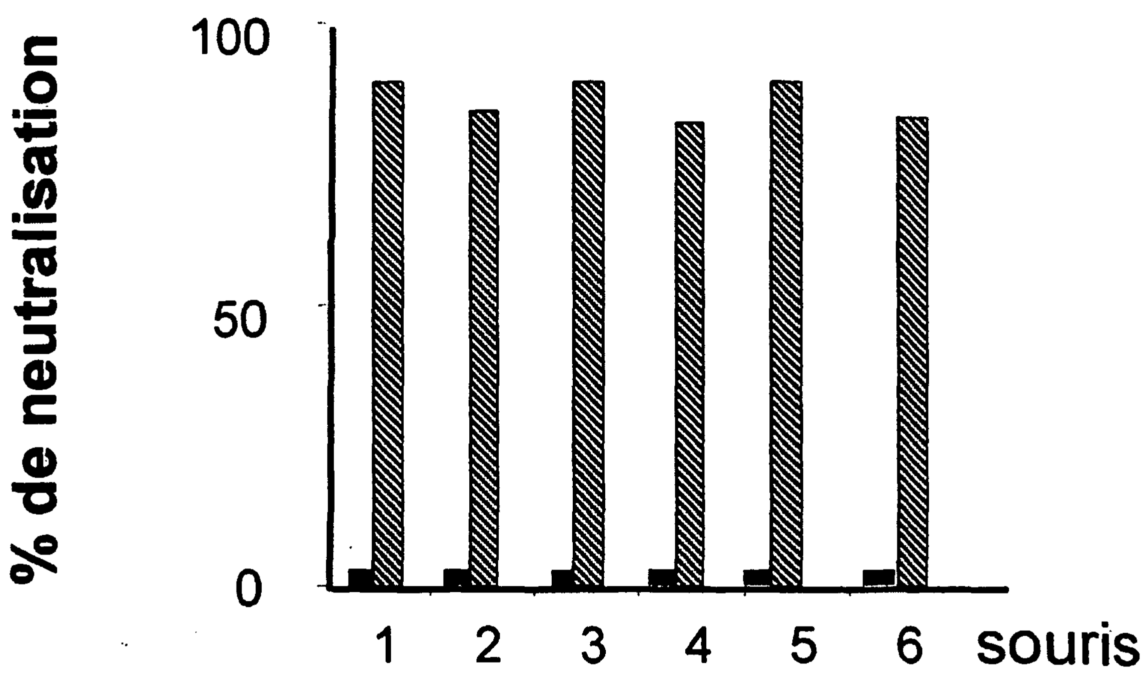


FIGURE 2

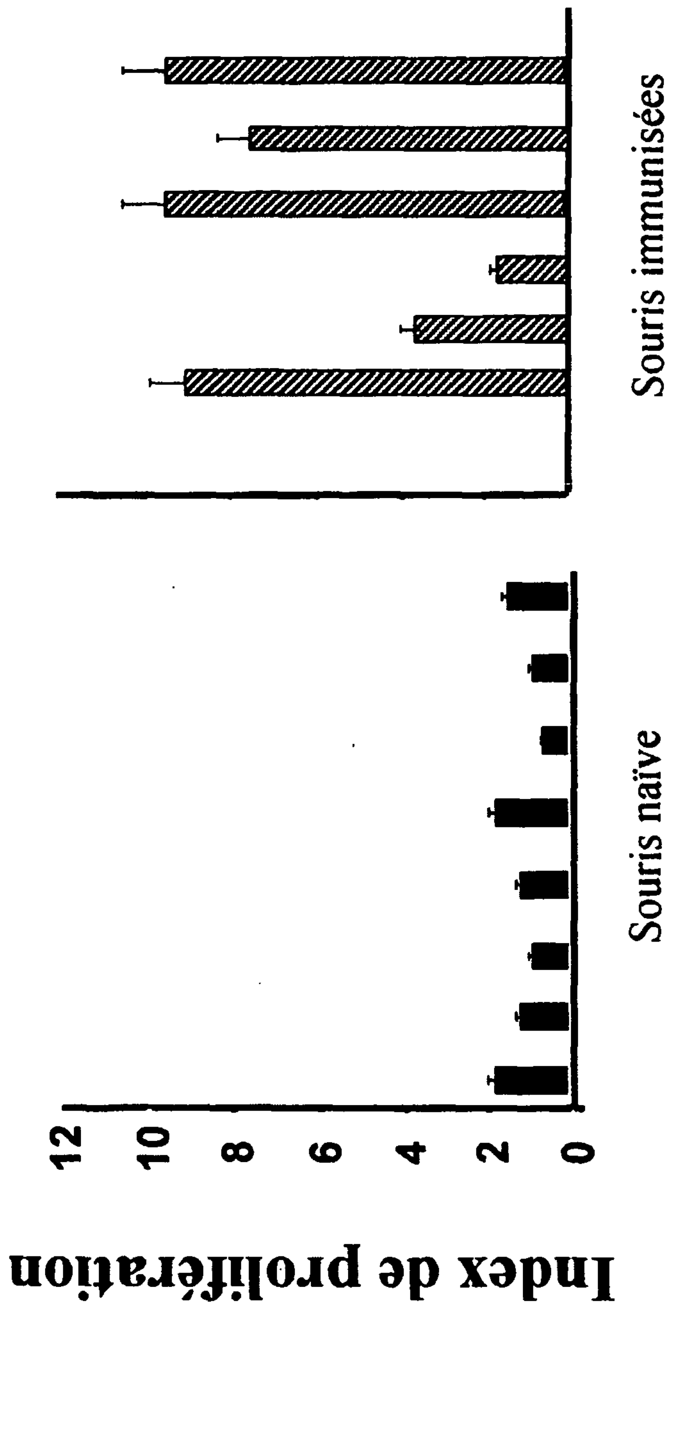
Index de prolifération
12
10
8
6
4
2
0
Souris naïve
Souris immunisées

FIGURE 3

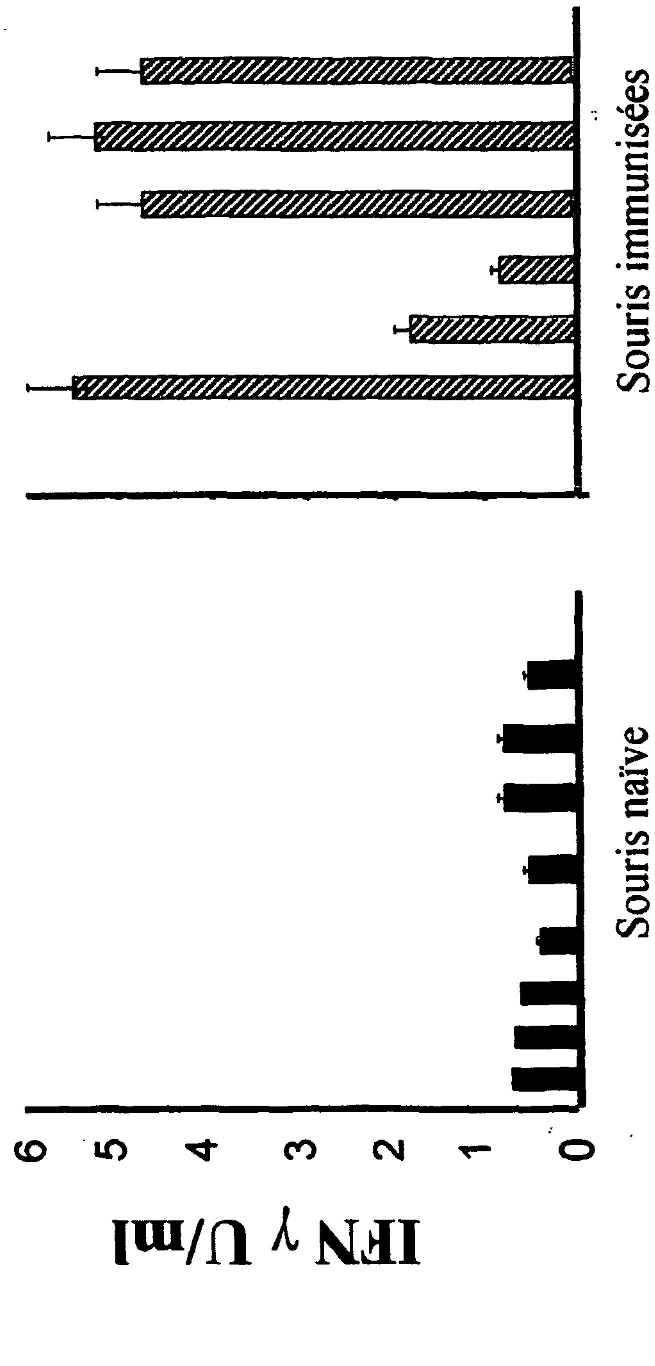


FIGURE 4

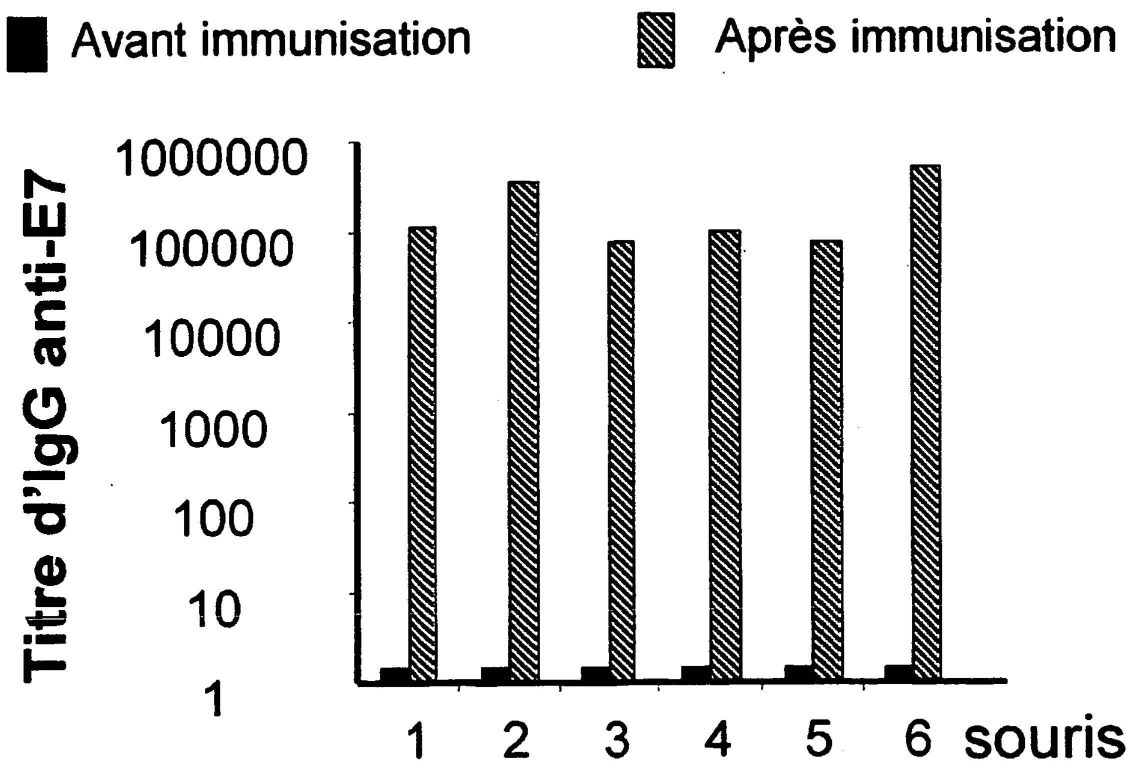


FIGURE 5

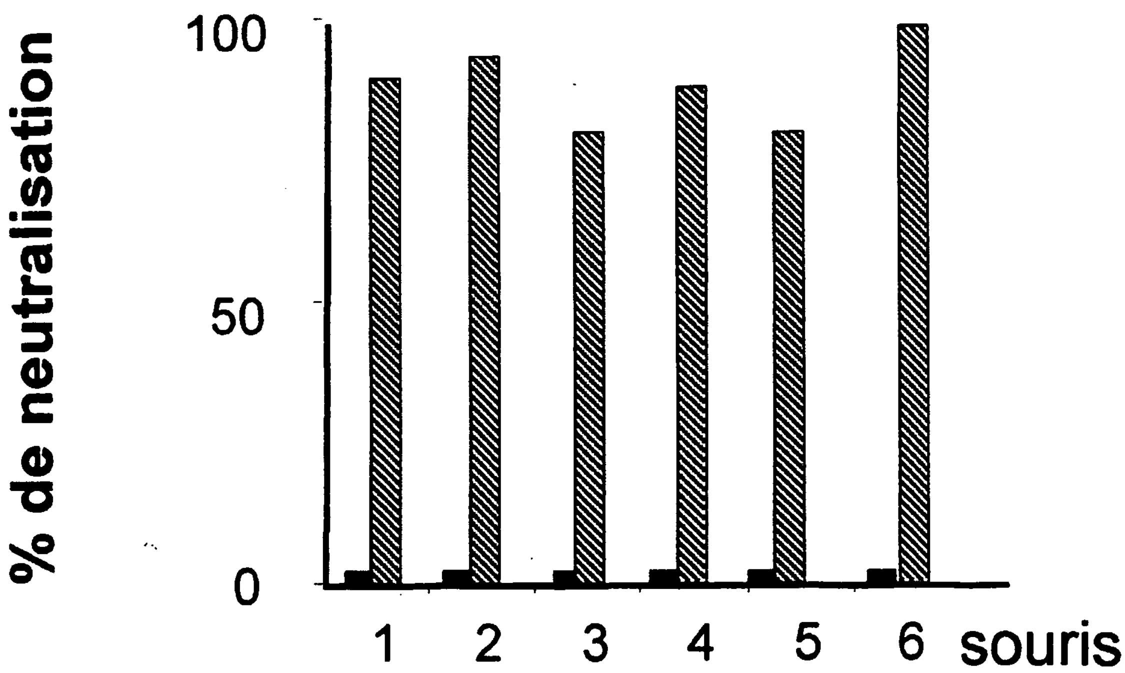


FIGURE 6

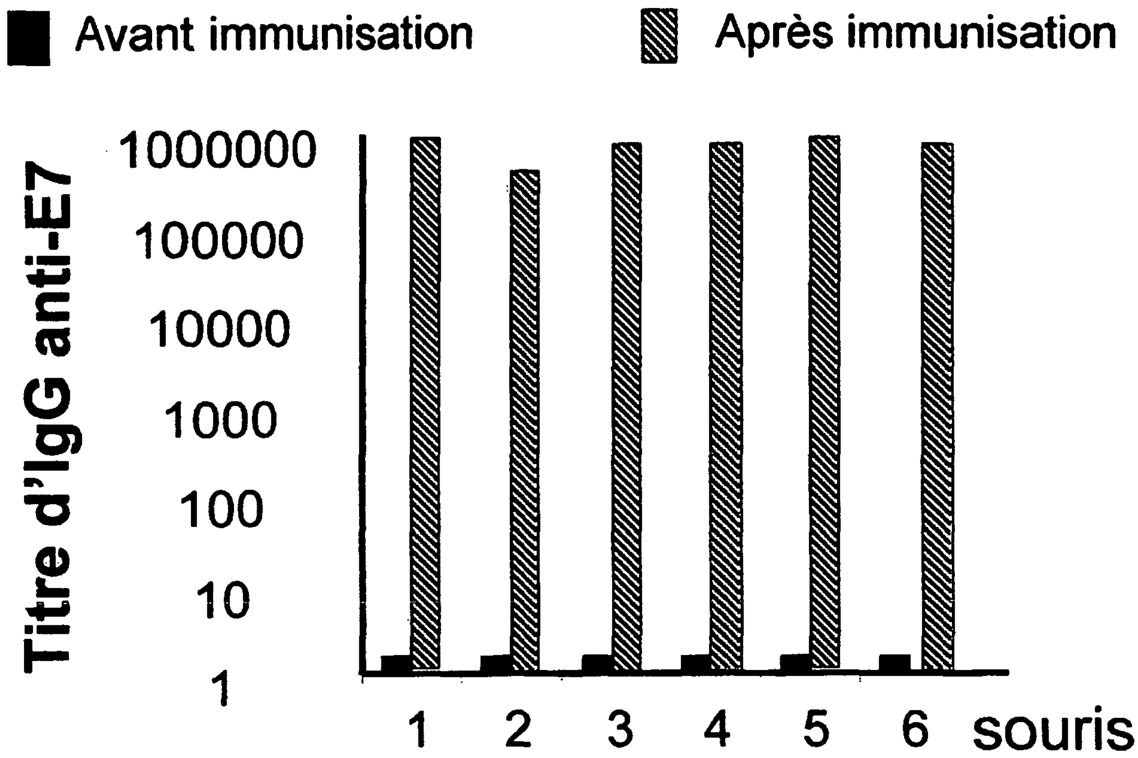


FIGURE 7

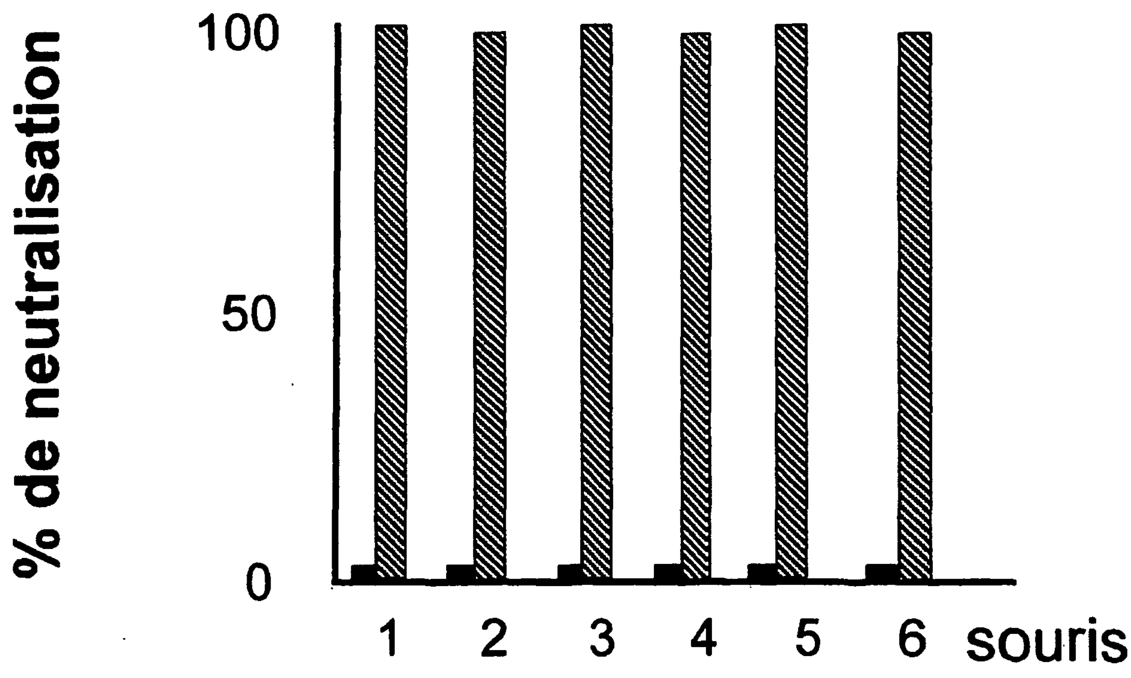


FIGURE 8

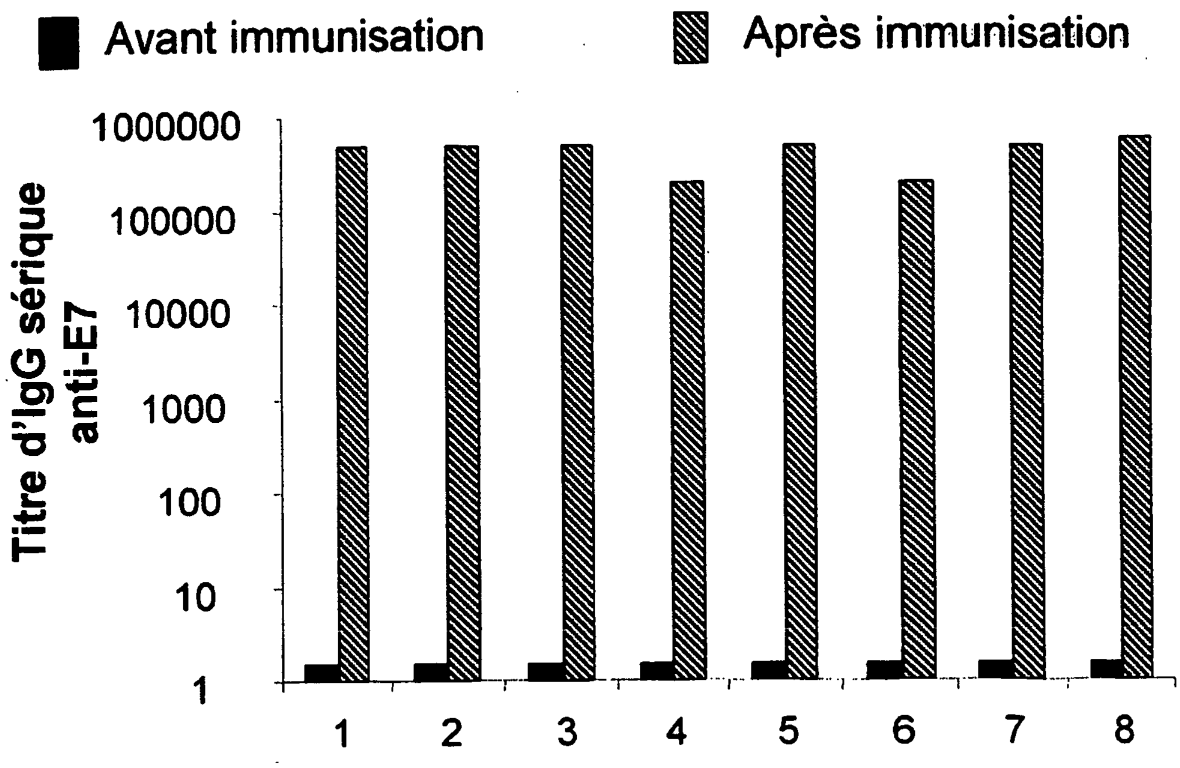


FIGURE 9

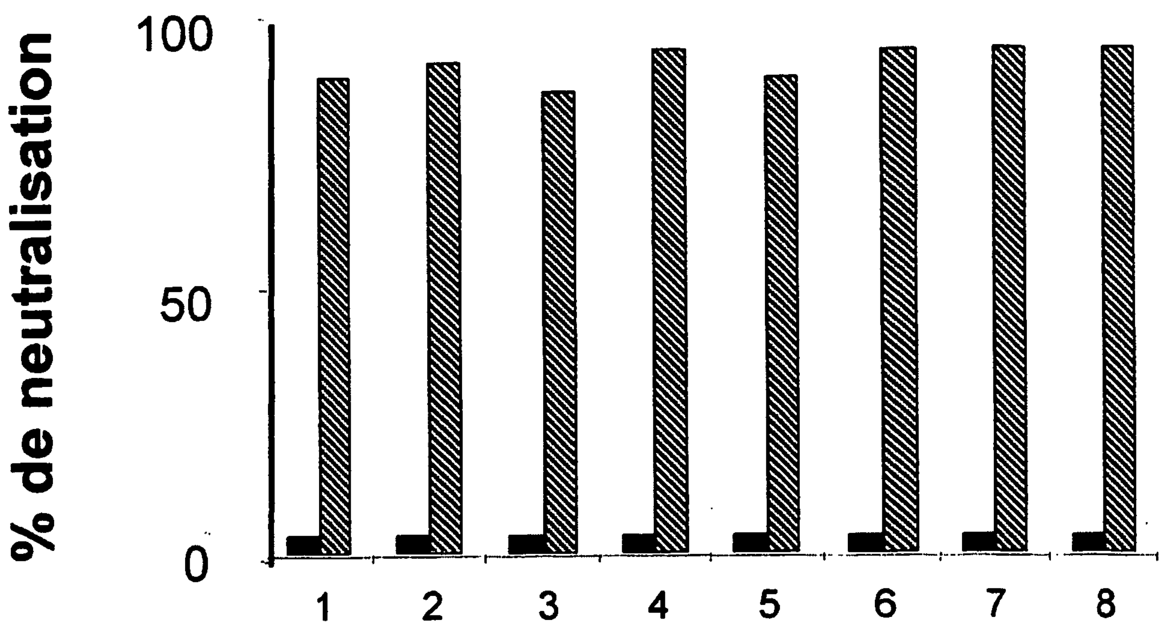


FIGURE 10

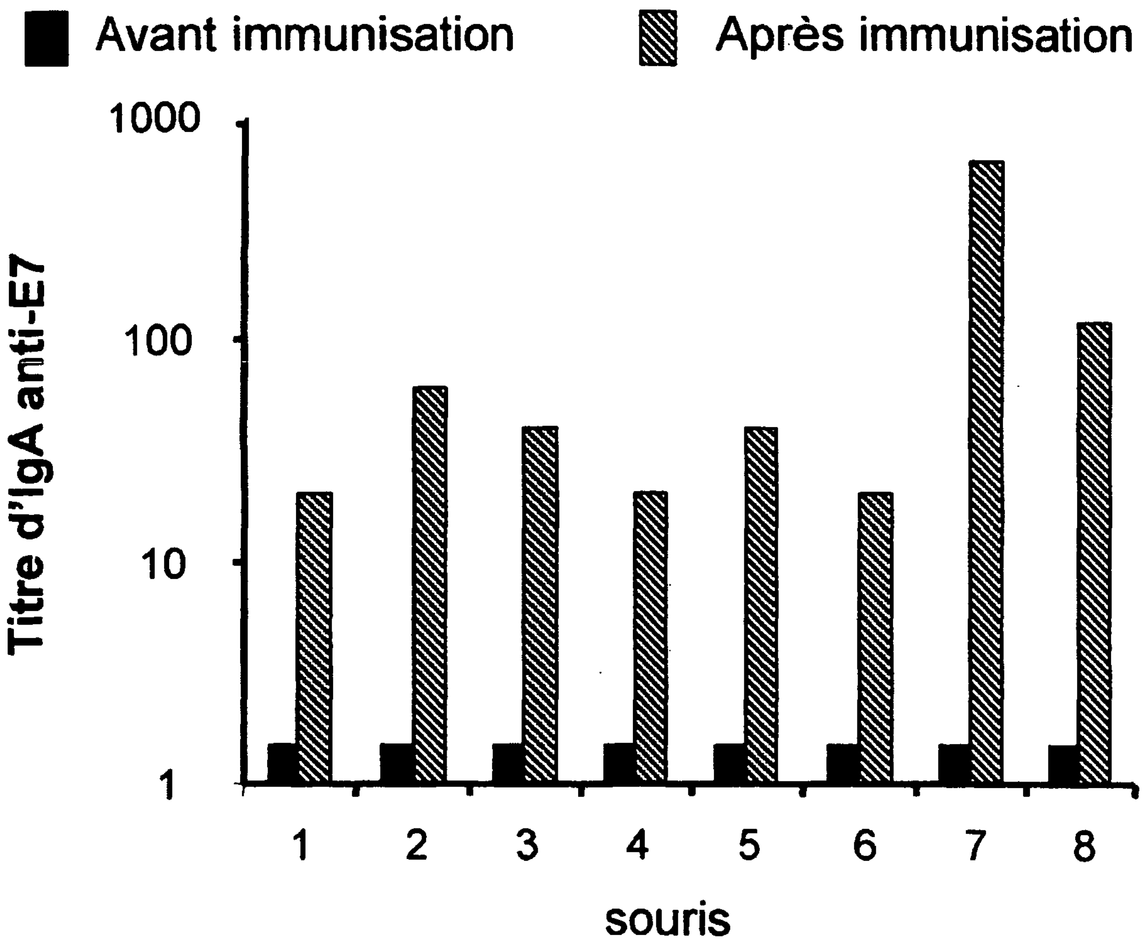
Avant immunisation
Après immunisation
1000
100
10
1
Titre d'IgA anti-E7
1
2
3
4
5
6
7
8
souris

FIGURE 11

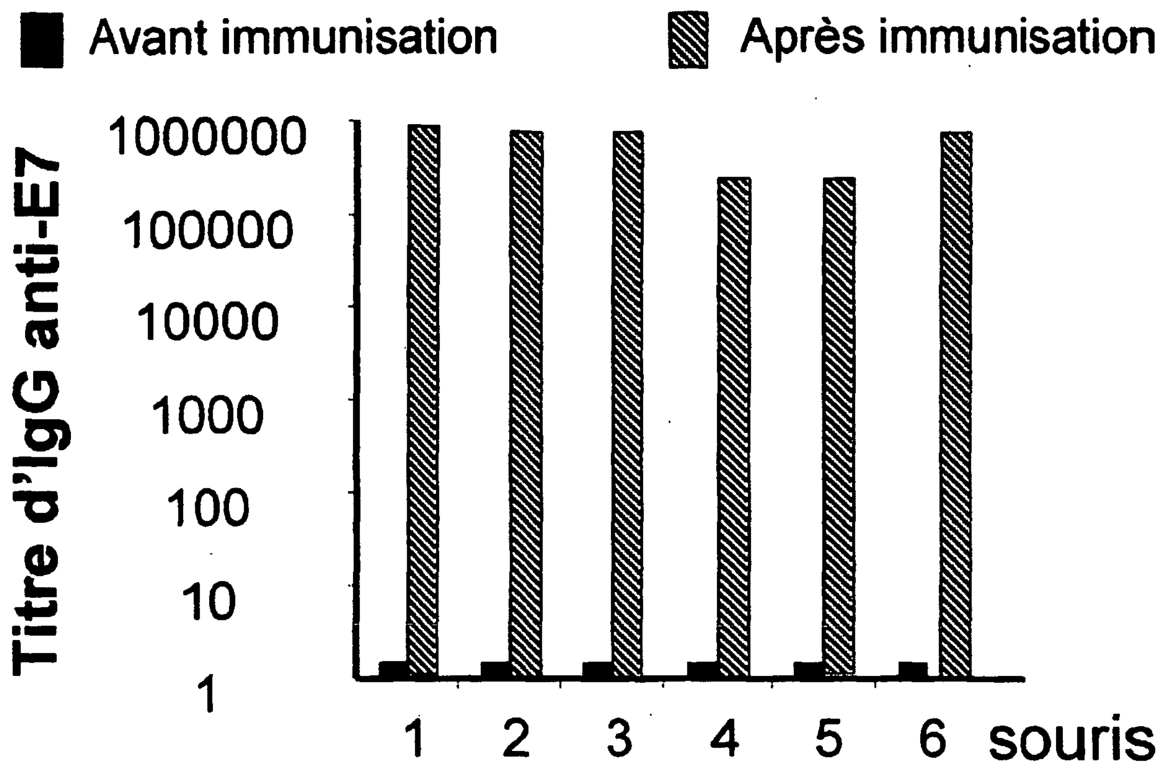


FIGURE 12

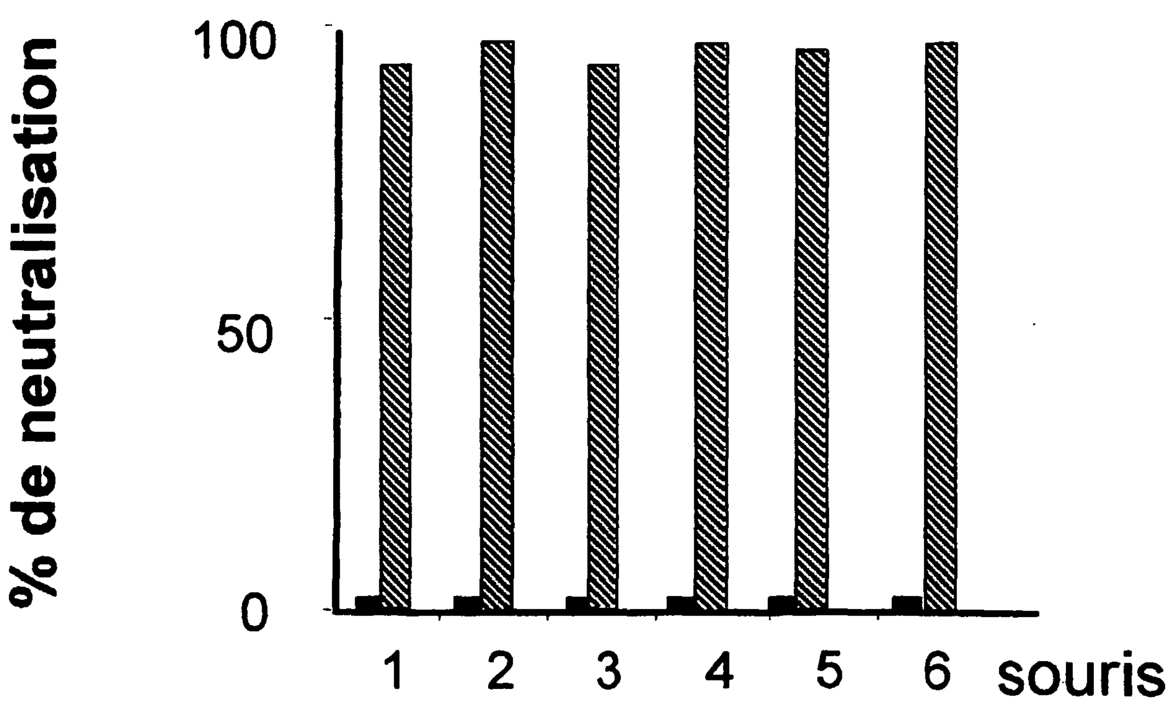


FIGURE 13

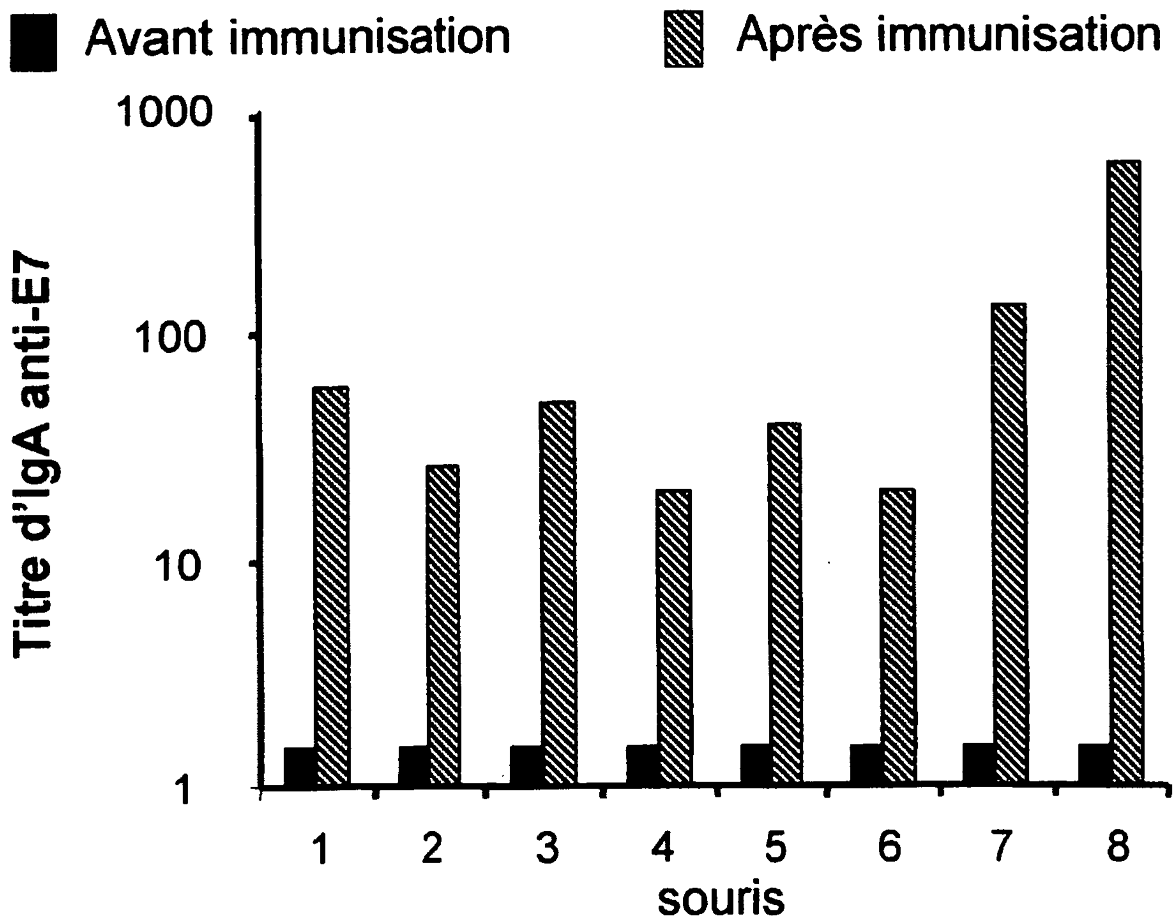
Avant immunisation
Après immunisation
1000
100
10
1
Titre d'IgA anti-E7
1
2
3
4
5
6
7
8
souris

FIGURE 14

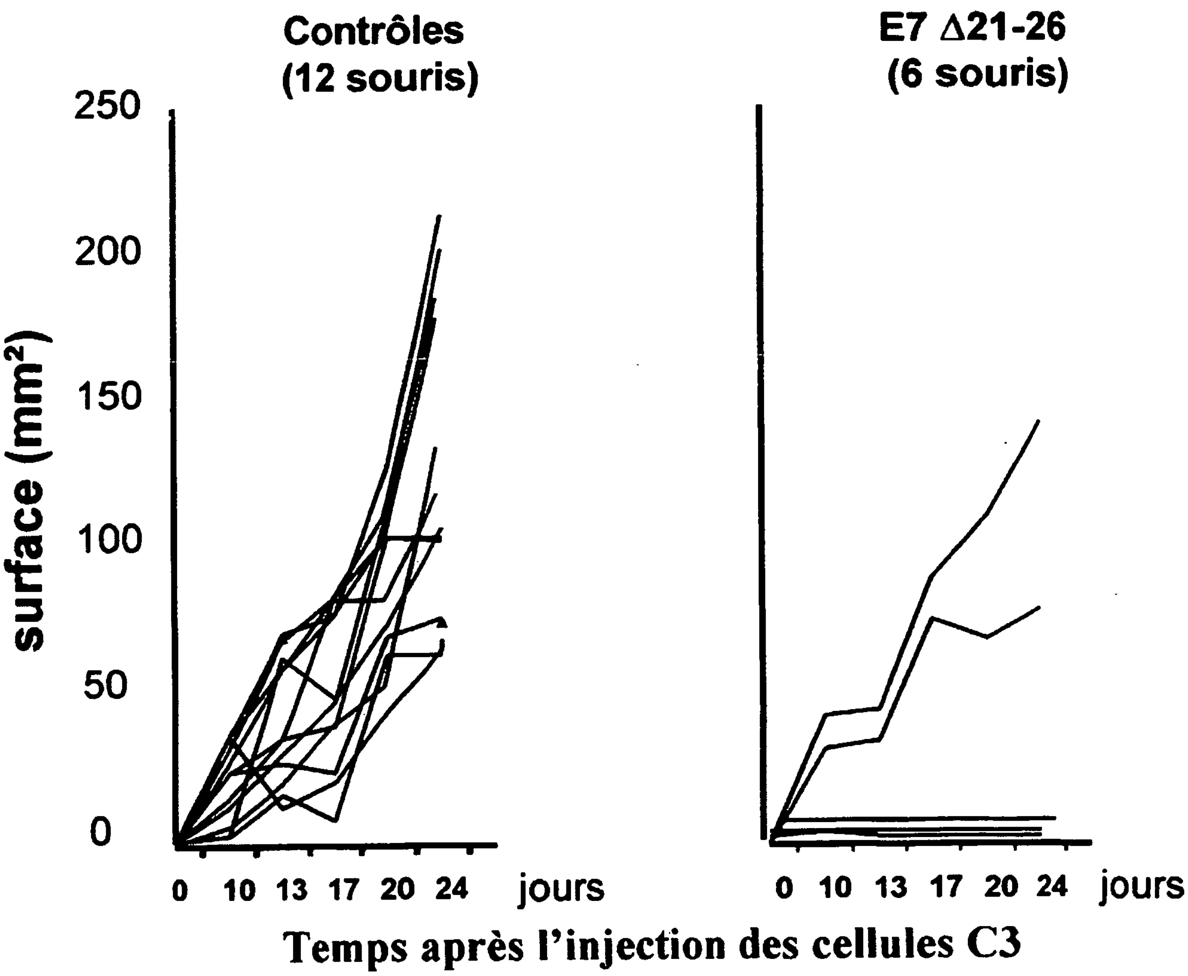
Contrôles
(12 souris)
E7 Δ21-26
(6 souris)
250
200
150
100
50
0
surface (mm²)
0 10 13 17 20 24 jours
0 10 13 17 20 24 jours
Temps après l'injection des cellules C3

FIGURE 15

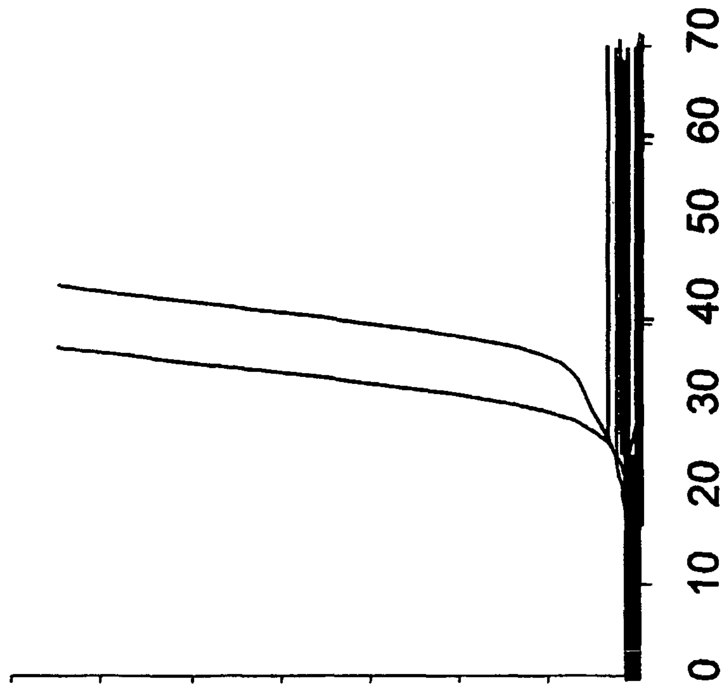


FIGURE 16B

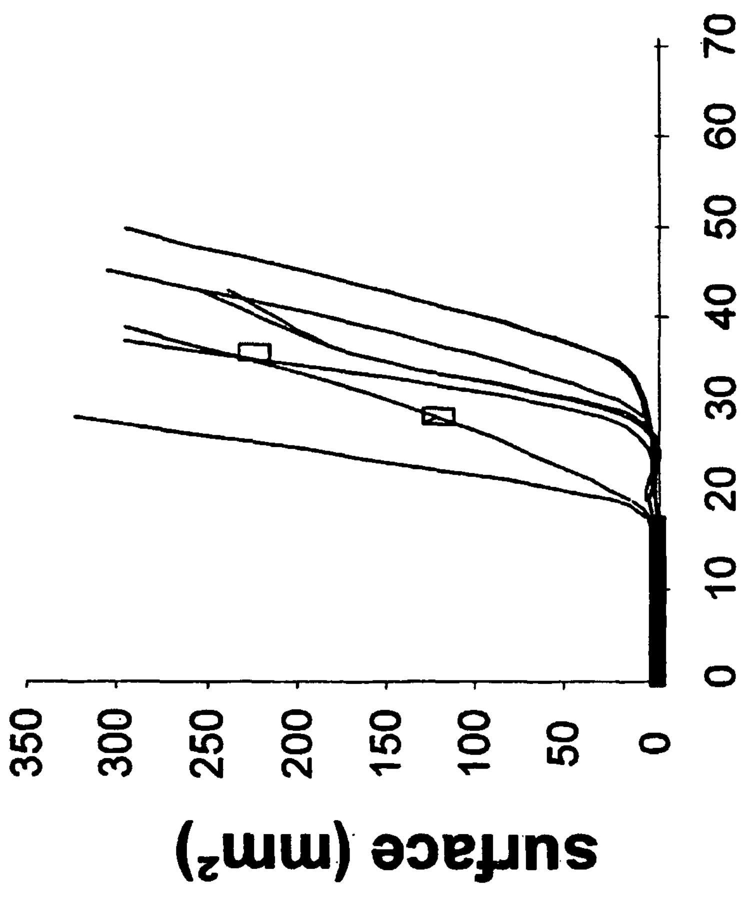


FIGURE 16A

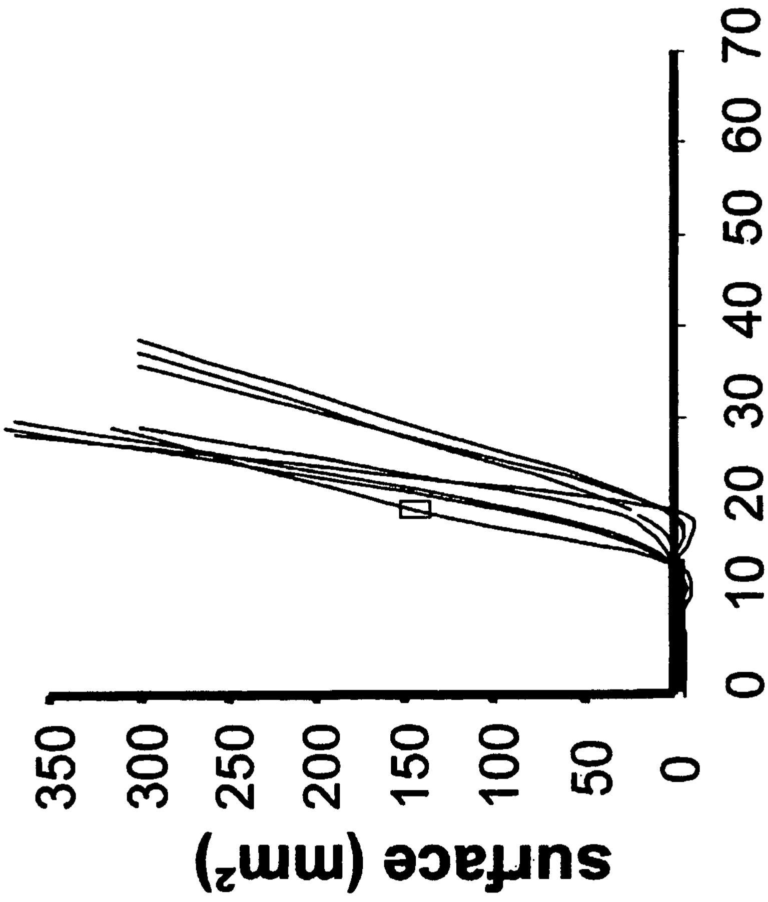


FIGURE 17A

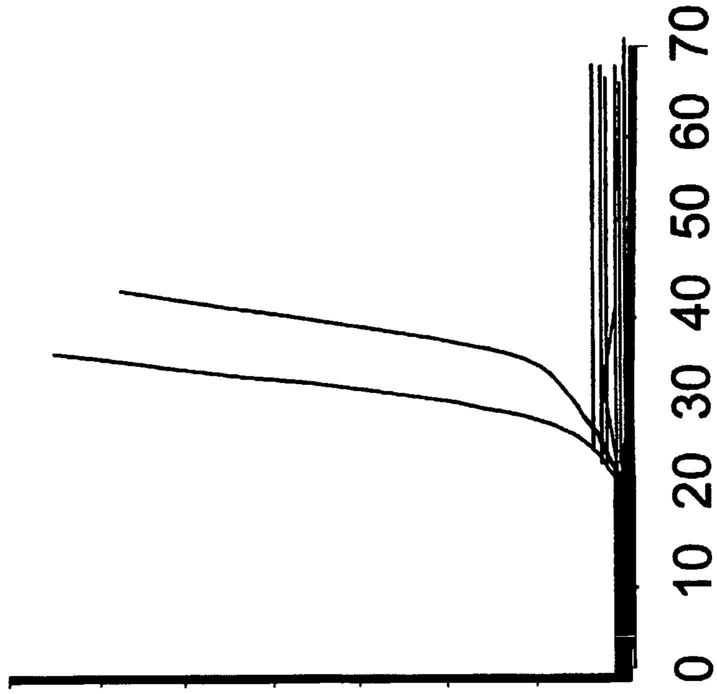


FIGURE 17B

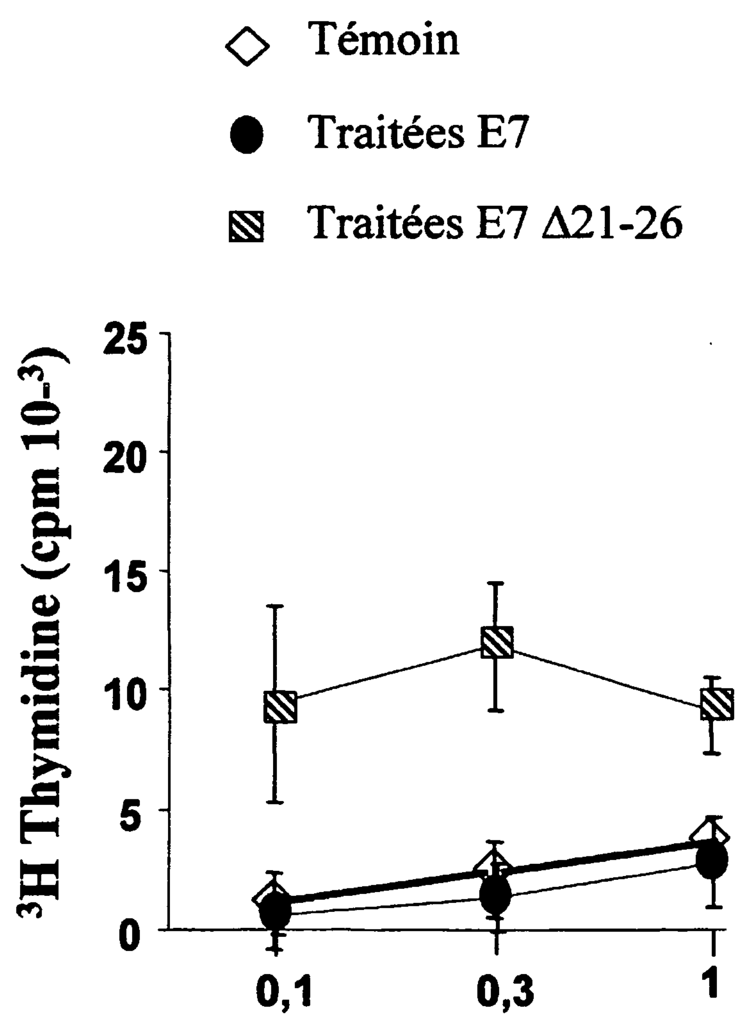
Témoin
Traitées E7
Traitées E7 Δ21-26
25
20
15
10
5
0
$^3$H Thymidine (cpm 10-$^3$)
0,1
0,3
1

FIGURE 18

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0003732 A
- WO 9504064 A
- FR 0110751
- WO 9511307 A
- FR 0205173


### Littérature non-brevet citée dans la description


- **D'ANNA et al.** *J. Natl. Cancer Inst.,* 2001, vol. 93 (24), 1843-1851
- **HERMANSON G.T.** Bioconjugate techniques. Academic Press, 1996, 239-242
- **SAMOSZUK M.K. et al.** *Antibody, Immunoconjugates Radiopharm,* 1989, vol. 2 (1), 37-46
- **FELTKAMP, M.C. ; H.L. SMITS ; M.P. VIERBOOM ; R.P. MINNAAR ; B.M. DE JONGH ; J.W. DRIJFHOUT ; J. TER SCHEGGET ; C.J. MELIEF ; W.M. KAST.** Vaccination with cytotoxic T lymphocyte epitope-containing peptide protects against a tumor induced by human papillomavirus type 16- transformed cells. *Eur.J.Immunol.,* 1993, vol. 23, 2242-2249
- **DE SMEDT, T. ; B. PAJAK ; E. MURAILLE ; L. LESPAGNARD ; E. HEINEN ; P. DE BAETSELIER ; J. URBAIN ; O. LEO ; M. MOSER.** Regulation of dendritic cell numbers and maturation by lipopolysaccharide in vivo. *J.Exp.Med.,* 1996, vol. 184, 1413-1424
- **SALLUSTO et al.** *Journal of Experimental Medicine,* 1994, vol. 179, 1109-18
- Current protocols in Molecular Biology. Wiley Interscience, 1989
- **AUCOUTURIER et al.** *Vaccine,* 2001, vol. 19, 2666-2672
- **BARAS et al.** *Infect. Immun.,* 1999, vol. 67, 2643-2648
- **BASAK et al.** *J. Pept. Sci.,* 1995, vol. 1 (6), 383-395
- **CHEN et al.** *Mol. Cell. Biol.,* 1987, vol. 7, 2745-2752
- **CRUZ , I. et al.** *Br. J. Cancer,* 1999, vol. 81, 881-889
- **EL SHERIF AM et al.** *J. Pathol,* 2001, vol. 195 (2), 179-185
- **FRANKEL et al.** *Cell,* 1988, vol. 55
- **FELTKAMP, M.C. ; H.L. SMITS ; M.P. VIERBOOM ; R.P. MINNAAR ; B.M. DE JONGH ; J.W. DRIJFHOUT ; J. TER SCHEGGET ; C.J. MELIEF ; W.M. KAST.** Vaccination with cytotoxic T lymphocyte epitope-containing peptide protects against a tumor induced by human papillomavirus type 16-transformed cells. *Eur.J.Immunol.,* 1993, vol. 23, 2242-2249
- **FRALEY.** *J. Biol. Chem.,* 1980, vol. 255, 10431
- **FLOTTE et al.** *Am. J. Respir. cell Mol. Biol.,* 1992, vol. 7, 349-356
- Nucleic acid hybridization. 1984
- **GERARD C M.** *Vaccine,* 2001, vol. 19, 2583-2589
- **GIANNINI et al.** *Clin. EXP. Immunol,* 1998, vol. 113 (2), 183-189
- **GIANNINI et al.** *Int. J. CANCER,* 2002, vol. 97 (5), 654-659
- **GOPAL.** *Mol. Cell. Biol.,* 1985, vol. 5, 1188-1190
- Oligonuclotide synthesis. DNA Cloning: A practical approach. MRL Press, Ltd, 1985, vol. I,II
- **GRAHAM et al.** *Virology,* 1973, vol. 52, 456-457
- **HOUBEN WEYL.** Meuthode der Organischen Chemie. Thieme, 1974, vol. 15-I,15-
- **HUYGEN et al.** *Nature Medicine,* vol. 2 (8), 893-898
- **HARLAND et al.** *J. Cell. Biol.,* 1985, vol. 101, 1094-1095
- **KIEFER H. et al.** *Biochemistry,* 1996, vol. 35 (50), 16077-16084
- **MCLAUGHLIN B A et al.** *Am. J. Hum. Genet,* 1996, vol. 59, 561-569
- **MERRIFIELD RB.** *Nature,* 1965, vol. 207 (996), 522-523
- **MERRIFIELD.** *Science,* 1965, vol. 150 (693), 178-185
- **MUDERSPACH L. et al.** *Clinical Cancer Research,* 2000, vol. 6, 3406-3416

- **N ICOLAU et al.** *Methods enzymol.,* 1987, vol. 149, 157-176
- **OSEN W et al.** *Vaccine,* 2001, vol. 19, 4276-4286
- **POTTER et al.** *Proc. Natl. Acad. sci. USA,* 1984, vol. 81 (22), 7161-7165
- **TUCKER J. ; GRISSHAMMER R.** *Biochem J.,* 1996, vol. 317 (3), 891-899
- **SAMBROOK J ; FRITSCH E F ; MANIATIS T.** Molecular Cloning: A Laboratory Manual. Cold Spring Harbor Laboratory, 1989
- **SAMULSKI et al.** *J. Virol.,* 1989, vol. 63, 3822-3828
- **SCHOELL W M J.** *Gynecologic Oncology,* 1999, vol. 74, 448-455
- **SEEDORF K.** *Virology,* 1985, vol. 145 (1), 181-185
- **SKAUGRUD et al.** *Biotechnol. Genet. Eng. Rev.,* 1999, vol. 16, 23-40
- **TACSON et al.** *Nature Medicine,* 1996, vol. 2 (8), 888-892
- **TUR-KASPA.** *Mol. Cell. Biol.,* 1986, vol. 6, 716-718

LISTE DE SEQUENCES

<110> NEOVACS

<120> Composition immunogène ou vaccinale non ummunosuppressive, comprenant une protéine E7 mutée du virus HPV-16

<130> NEOVACS - N729-PCT

<140>

<141>

<150> FR0205173
<151> 2002-04-24

<160> 4

<170> PatentIn Ver. 2.1

<210> 1
<211> 92
<212> PRT
<213> Séquence artificielle

<220>
<223> Description de la séquence artificielle:E7-delta-21-26

<400> 1

```
Met His Gly Asp Thr Pro Thr Leu His Glu Tyr Met Leu Asp Leu Gln
 1               5                  10                  15

Pro Glu Thr Thr Gln Leu Asn Asp Ser Ser Glu Glu Glu Asp Glu Ile
            20                  25                  30

Asp Gly Pro Ala Gly Gln Ala Glu Pro Asp Arg Ala His Tyr Asn Ile
        35                  40                  45

Val Thr Phe Cys Cys Lys Cys Asp Ser Thr Leu Arg Leu Cys Val Gln
    50                  55                  60

Ser Thr His Val Asp Ile Cys Thr Leu Glu Asp Leu Leu Met Gly Thr
65                  70                  75                  80

Leu Gly Ile Val Cys Pro Ile Cys Ser Arg Lys Pro
                85                  90
```

<210> 2
<211> 276
<212> ADN
<213> Séquence artificielle

<220>
<223> Description de la séquence artificielle:E7-delta-21-26

<400> 2

```
catggagata cacctacatt gcatgaatat atgttagatt tgcaaccaga gacaactcaa 60

ttaaatgaca gctcagagga ggaggatgaa atagatggtc cagctggaca agcagaaccg 120
gacagagccc attacaatat tgtaaccttt tgttgcaagt gtgactctac gcttcggttg 180
tgcgtacaaa gcacacacgt agacattcgt actttggaag acctgttaat gggcacacta 240
ggaattgtgt gccccatctg ttctcagaaa ccataa                           276
```

<210> 3
<211> 98
<212> PRT
<213> Human papillomavirus type 16

<400> 3

```
Met His Gly Asp Thr Pro Thr Leu His Glu Tyr Met Leu Asp Leu Gln
 1               5                  10                  15

Pro Glu Thr Thr Asp Leu Tyr Cys Tyr Glu Gln Leu Asn Asp Ser Ser
            20                  25                  30

Glu Glu Glu Asp Glu Ile Asp Gly Pro Ala Gly Gln Ala Glu Pro Asp
        35                  40                  45

Arg Ala His Tyr Asn Ile Val Thr Phe Cys Cys Lys Cys Asp Ser Thr
    50                  55                  60

Leu Arg Leu Cys Val Gln Ser Thr His Val Asp Ile Cys Thr Leu Glu
65                  70                  75                  80

Asp Leu Leu Met Gly Thr Leu Gly Ile Val Cys Pro Ile Cys Ser Arg
                85                  90                  95

Lys Pro
```

<210> 4
<211> 294
<212> ADN
<213> Human papillomavirus type 16

<400> 4

```
catggagata cacctacatt gcatgaatat atgttagatt tgcaaccaga gacaactgat 60
ctctactgtt atgagcaatt aaatgacagc tcagaggagg aggatgaaat agatggtcca 120
gctggacaag cagaaccgga cagagcccat tacaatattg taacctttg ttgcaagtgt 180
gactctacgc ttcggttgtg cgtacaaagc acacacgtag acattcgtac tttggaagac 240
ctgttaatgg gcacactagg aattgtgtgc cccatctgtt ctcagaaacc ataa       294
```